(19)

**Europäisches Patentamt**
**European Patent Office**
**Office européen des brevets**

(11) **EP 2 832 373 B1**

(12) **EUROPEAN PATENT SPECIFICATION**

(45) Date of publication and mention
of the grant of the patent:
**12.04.2017 Bulletin 2017/15**

(51) Int Cl.:
*A61K 47/69* (2017.01)   *A61P 29/00* (2006.01)
*A61P 35/00* (2006.01)   *A61P 9/10* (2006.01)
*A61K 9/127* (2006.01)   *A61K 9/51* (2006.01)
*A61K 31/40* (2006.01)   *A61K 38/08* (2006.01)

(21) Application number: **13003858.1**

(22) Date of filing: **02.08.2013**

(54) **Liposome for blocking site-specifically chemokine-related inflammatory processes in vascular diseases and metastasis**

Liposom zur Blockierung eines standortspezifischen chemokinassoziierten entzündlichen Prozesses bei Gefäßerkrankungen und Metastasen

Liposome permettant de bloquer des processus inflammatoires liés aux chimiokines spécifiques à un site dans des maladies vasculaires et la métastase

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.02.2015 Bulletin 2015/06**

(73) Proprietors:
• **Experimentelle Pharmakologie & Onkologie Berlin Buch**
  **13125 Berlin (DE)**
• **Institute of Cellular Biology and Pathology 'Nicolae Siminescu'**
  **Bucharest (RO)**
• **University Politehnica of Bucharest**
  **060042 Bucharest (RO)**
• **University of Bonn**
  **53121 Bonn (DE)**
• **Istanbul University**
  **34452 Beyazit, Istanbul (TR)**
• **University of Zurich**
  **8057 Zürich (CH)**

(72) Inventors:
• **Bendas, Gerd**
  **53225 Bonn (DE)**
• **Schlesinger, Martin**
  **50935 Köln (DE)**
• **Borsig, Lubor**
  **8192 Glattfelden (CH)**
• **Cevher, Erdal**
  **Uskudar, Istanbul (TR)**

• **Pabuccuoglu, Saadet Kevser**
  **Instanbul (TR)**
• **Gok, M. Koray**
  **Instambul (TR)**
• **Mihaly, Maria**
  **060573 Bucharest (RO)**
• **Enachescu, Marian**
  **Bucharest (RO)**
• **Calin, Manuela Calin**
  **023791 Bucharest (RO)**
• **Simionescu, Maya**
  **050535 Bucharest (RO)**
• **Zeisig, Reinhard**
  **10245 Berlin (DE)**
• **Hoffmann, Annika**
  **16359 Biesenthal (DE)**

(74) Representative: **Baumbach, Friedrich Patentanwalt**
  **Robert-Rössle-Strasse 10**
  **13125 Berlin (DE)**

(56) References cited:
**EP-A1- 2 226 083**   **WO-A1-93/20102**
**WO-A1-2009/065181**   **US-A1- 2010 166 739**
**US-A1- 2011 098 212**   **US-A1- 2012 171 121**
**US-A1- 2012 219 602**   **US-B1- 6 451 842**

EP 2 832 373 B1

- MOREE W J ET AL: "Potent antagonists of the CCR2b receptor. Part 3: SAR of the (R)-3-aminopyrrolidine series", BIOORGANIC AND MEDICINAL CHEMISTRY LETTERS 20080315 GB, vol. 18, no. 6, 15 March 2008 (2008-03-15) , pages 1869-1873, XP002716534, ISSN: 0960-894X
- FABIENNE DANHIER ET AL: "PLGA-based nanoparticles: An overview of biomedical applications", JOURNAL OF CONTROLLED RELEASE, vol. 161, no. 2, 4 February 2012 (2012-02-04), pages 505-522, XP055051719, ISSN: 0168-3659, DOI: 10.1016/j.jconrel.2012.01.043
- ERIC SIMONE ET AL: "Targeted delivery of therapeutics to endothelium", CELL AND TISSUE RESEARCH, SPRINGER, BERLIN, DE, vol. 335, no. 1, 25 September 2008 (2008-09-25), pages 283-300, XP019658519, ISSN: 1432-0878
- BRECHT A ET AL: "Relaxin inhibits early steps in vascular inflammation", REGULATORY PEPTIDES, ELSEVIER SCIENCE BV, NL, vol. 166, no. 1-3, 17 January 2011 (2011-01-17), pages 76-82, XP027561030, ISSN: 0167-0115 [retrieved on 2010-12-15]
- PREISS D J ET AL: "Vascular cell adhesion molecule-1: a viable therapeutic target for atherosclerosis?", INTERNATIONAL JOURNAL OF CLINICAL PRACTICE APR 2007, vol. 61, no. 4, April 2007 (2007-04), pages 697-701, XP002716535, ISSN: 1368-5031

## Description

### FIELD OF THE INVENTION

The present invention relates to the pharmaceutical field, in particular to specific drug delivery. The invention provides a product for blocking site-specifically chemokine-related inflammatory processes in vascular diseases and tumor cell metastasis employing vascular targeted nanocarriers.

### BACKGROUND OF THE INVENTION

Atherosclerotic plaque formation and cancer cell metastasis have in common an inflammatory process and a specific repertoire of chemokines and cell adhesion molecules expressed by endothelial cells (EC) that attract particular immune cells (i.e. leukocytes) to the developing atheroma or enable tumor cells to invade and to metastasize within the tissues. Atherosclerosis is a chronic inflammatory disorder characterized by lipid accumulation within the arterial wall [Simionescu M., Arterioscler. Thromb. Vasc. Biol. 27 (2007) 266-74]. The progression of atherosclerotic plaque is driven by leukocyte infiltration, a process highly regulated by chemokines. Chemokines are small, highly specialized polypeptides that function as modulators of cellular traffic through their interactions with G-Proteincoupled receptors at a spatially defined site [Rot, A., von Andrian U.H., Annu. Rev. Immunol. 22 (2004) 891-928]. Chemokines bind to glycosaminoglycans (GAGs) present on the membrane of endothelial cells and guide leukocyte entry into the arterial wall.

Several lines of evidence suggest that inflammation enables tumor cells to invade and metastasize [Mantovani A. et al., Nature 454 (2008) 436-44]. Chemokines and their receptors have been shown to contribute to metastasis by directly affecting cancer cells, or by modulation of the tumor microenvironment. Previously, it was shown that the recruitment of inflammatory leukocytes to the metastatic sites profoundly affects metastatic progression [Laubli H. et al., Blood 114 (2009) 4583-91]. The local expression of chemokines (f. e. CCL5, CCL2 etc.) and the up-regulation of adhesion molecules (selectins, integrins) contribute to the creation of a metastatic niche. The inflammatory-like activation of the metastatic microenvironment stimulates the recruitment of bone marrow-derived cells of myeloid lineage that contribute to metastatic progression by several mechanisms including remodeling of the stroma, enabling invasiveness, and immune-suppression.

Since chemokines are known to be critically involved in the development of chronic inflammatory-associated diseases (such as atherosclerosis) and has been shown to promote metastasis in cancers of various origins, the intervention on the chemokine system opens new avenues in the prevention and treatment of these disorders. The functional manipulation of the chemokine system such as inhibition of chemokine/chemokine receptor interactions by using of chemokine antagonists (CA) or chemokine receptor antagonists (CRA) could constitute an important therapeutic option by preventing the accumulation of inflammatory immune cells that drives atherogenesis or the metastasis of tumor cells. Systemic interventions in the function of one or more chemokine have a high risk of side effects, such as impaired host defense against pathogens. Experimental and clinical testing of potential inhibitors of chemokines and their receptors for the treatment of inflammatory diseases revealed detrimental immunological effects [Proudfoot A.E., Power C.A., Schwarz, M.K. Expert Opin Investig Drugs 19 (2010) 345-55].

Several pharmaceutical compositions comprising chemokine antagonists or chemokine receptor antagonists for the treatment of diseases associated with inflammatory diseases are discussed in the state of the art:

US 6,451,842 B1 and Moree W.J. et al. [Bioorganic and medical chemistry letters, 18 (6): 1869-1873] disclose MCP (metoclopramide)-1/CCR2 antagonists, in particular teijin, and their potential use in the therapeutic treatment of inflammatory diseases like atherosclerosis.

WO 93/20102 A1 discloses the D-Ala-peptid-T-amid (DAPTA) peptide for the treatment of inflammation, in particular caused by cancer.

Therefore, the development of new and innovative therapeutic approaches to manipulate selectively specific chemokine(s) function in a pathophysiological context may have few or no adverse effects on the immune system. The local delivery of CA or CRA at the specific sites may constitute a better strategy to slow down or interrupt a confined inflammatory process.

So far, the prior art provides several colloidal systems for the general delivery of therapeutic agents to cells, usually in the form of liposomes, polymeric nanospheres, micelles, polymer-based hydrogels and oil-in-water emulsions. It could be shown that polymeric PLGA nanoparticles increase the circulation life time, avoid the chemical or enzymatic degradation and the rapid elimination of therapeutic agents (see f.e.: Danhier F. et al. [J. of controlled release, 161 (2): 505-522] and Simone E. et al. [Cell and tissue research, 335 (1): 283-300]). Further it is proposed in the art as illustrated by Danhier et al. and Simone E. et al., that the disclosed PLGA nanoparticles can be used for the treatment of inflammatory diseases.

Moreover, Brecht A. et al. (Regulatory Peptides, 166 (1-3), 76-82] or Preiss D.J. et al. [Int. J. of Clinical Practise, 61 (4): 697-701] disclose that VCAM-1 targeting will be beneficial in the case of treatments of inflammatory processes. Despite huge advantages, the targeted carrier technology is not without difficulties. Injected carriers have found limited applications due to their short vascular circulation lifetime. According to their size and surface characteristics, many particles are eliminated within short periods after their injection. Consequently, the rapid sequestration of intravenously injected carrier systems is problematic for a prolonged circulation and efficient targeting of the drug carrier. The biggest challenge is to stimulate accumulation of the drug loaded carrier at the target site. Therefore the nanocarriers should be supplied with specific probes capable for binding the target cell. Such nanocarriers need to have longevity and target recognition. So far, this problem could not be solved satisfyingly and an efficient "long circulating" system for targeting drugs in a manner to manipulate selectively specific chemokine(s) function is not yet available from the state of the art [Muro S. J Control Release 164 (2012) 125-37.; Kumar P, Gulbake A, Jain SK. Crit Rev Ther Drug Carrier Syst. 29 (2012) 355-419; Swaminathan J, Ehrhardt C. Expert Opin Drug Deliv. 9 (2012) 1489-503].

## AIMS AND OBJECTIVES OF THE INVENTION

The general aim of the invention is to find an alternative therapeutic way, in particular to develop specifically designed nanocarriers to be used as long circulating vehicles for targeted drug delivery to inflammatory sites.

In particular, the aim is to design specific *in vivo* long stable nanocarriers carrying chemokine antagonists (CA) or chemokine receptor antagonists (CRA) to inhibit the transmigration of leukocytes (in atherosclerosis) or of tumor metastatic cells (in cancer).

## DESCRIPTION OF THE INVENTION

The invention is implemented according to claim 1, 7 and 8, the subclaims are preferred alternatives.

The size and surface properties of the nanocarriers are of crucial importance in achieving controlled drug delivery. Ideally, nanocarriers should be small biodegradable particles, with good loading capacity, prolonged circulation and ability to accumulate in required areas. These requirements are reasonable well-met with the nanocarriers described in the present invention.

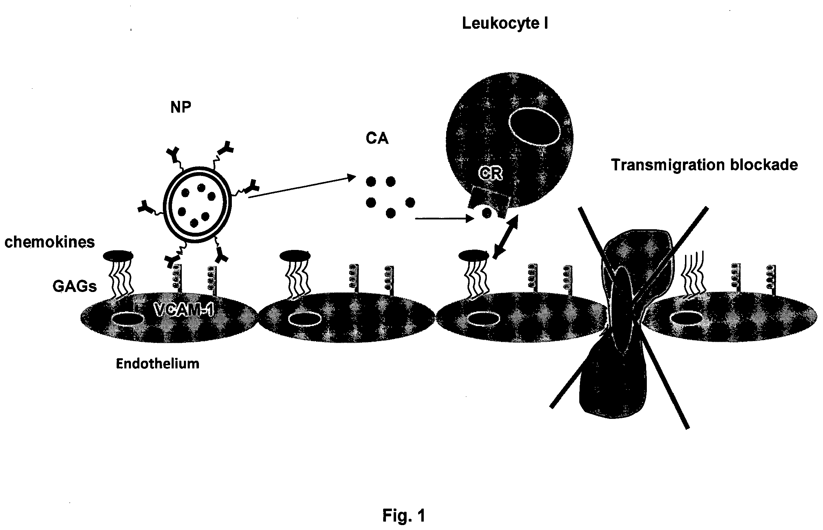

In summary, the present disclosure provides two types of targeted nanocarrier (NC) with encapsulated chemokine antagonists (CA) or chemokine receptor antagonists (CRA) which are stabilised target-sensitive liposomes (TSL) covered by the claims and polymeric nanoparticles (PNP) which are not covered by the claims. Both types of nanocarriers have attached on their surface monoclonal antibodies or peptides that recognize VCAM-1 expressed by activated endothelial cells (EC). After binding of the nanocarrier to the VCAM-1 expressing EC, the encapsulated CA or CRA are released and will block the interaction between chemokine and chemokine receptors. This will lead to an inhibition of transmigration of leukocytes (in atherosclerosis) or of tumor metastatic cells (in cancer) (see figure 1).

These and other objects of the present invention will be disclosed in more detail in the following sections and also by means of the embodiments.

The claimed nanocarriers for targeted drug delivery to inflammatory sites are characterized in that the nanocarrier are

- liposomes comprising dioleoylphosphatidylethanolamine (DOPE) stabilized in a bilayer with dioleoylphospatidicacid (DOPA) or 1,2-dipalmitoyl-*sn*-glycero-3-phospho-(1'-*rac*-glycerol) (DPPG)
- and wherein said nanocarrier comprising a peptide or antibody which specifically binds to endothelial cell adhesion molecule VCAM-1
- and wherein said nanocarrier encapsulate chemokine antagonists (CA) or chemokine receptor antagonists (CRA).

The liposomes comprises preferably polyethyleneglycol (PEG) modified phospholipids. The VCAM-1 specific peptide is coupled to the distal end of the polyethyleneglycol.

The Nanocarriers are further characterized, in that Chemokine (C-C motif) Ligand 2 Antagonist (CCL2-Antagonist) or Chemokine (C-C motif) Ligand 5 Antagonist (CCL-5-Antagonist) is encapsulated, preferably the Chemokine Receptor 2 Antagonist Teijin or the Chemokine Receptor 5 Antagonist DAPTA.

The particle size of the nanocarriers is between 50 and 500 nm, preferably between 70 and 200 nm, more preferably 200 nm.

The Nanocarriers according to the Invention are used in blocking cancer cell metastasis or in blocking inflammatory processes in vascular diseases.

First type of novel nanocarrier: target-sensitive Liposomes (TSL)

Liposomes are closed spherical vesicles consisting of a lipid bilayer containing phospholipids that encapsulates an internal aqueous environment in which drugs can be stored. Since long times their use have been well known as vesicles for the delivery of active ingredients.

Liposomes can be classified in multilamellar vesicles (MLV) with several lipid bilayers or in large unilamellar vesicles (LUV) with only one lipid bilayer.

The basic component for TSL (LUV) in the present invention comprises dioleoylphosphatidylethanolamine (DOPE, 60-90%, preferably 80%) that is stabilised in a bilayer with dioleoylphospatidicacid (DOPA, 10-40%, preferably 20%) or 1,2-dipalmitoyl-sn-glycero-3-phospho-(1'-rac-glycerol) (DPPG, 10-40%, preferably 20%). According to the present invention, the liposome must comprise polyethyleneglycol (PEG) modified phospholipids, which are essential for long circulating liposomes after i.v. injection *in vivo.*

This disclosed composition of target-sensitive liposomes guarantees the stability of the targeted carrier system in blood until it reaches the target on the surface of activated endothelial cells.

It turned out that VCAM-1, an immunoglobulin-like transmembrane glycoprotein expressed on inflammatory (activated) endothelium or vascular sites of metastatic progression, is an excellent target for binding the nanocarriers to the endothelial cells. Therefore, VCAM-1 binding antibodies (or antibody fragments) or a linear peptide preferably with the amino acid sequence *VHPKQHR* or a peptide sequence containing this sequence is coupled to the liposome surface using membrane anchors.

In particular, the VCAM-1 binding antibody, antibody fragment or peptide is coupled to the distal ends of phospholipid anchored PEG. The evaluation of coupling efficiency was done by HPLC or dinitrobenzoic acid assay.

Liposomes were characterized with respect to their size and physical stability as a function of time, temperature and the presence of serum using fluorescent markers with self-quenching properties, such as entrapped calcein or 6-carboxyfluorescein. The fluorescence resulting from liposome bilayer destabilization has been measured by fluorimetry.

Environmental scanning electron microscopy (SEM) and atomic force microscopy (AFM) have been employed for morphological characterization since they do not modify the liposome preparations and will allow the differentiation of individual structures for each component of the formulation.

The liposomal disruption after binding to the EC surface has been simulated using a SAW biosensor system and atomic force microscopy.

Second type of novel nanocarrier: Polymeric Nanoparticles (PNP)

This second type of nanocarrier represents a nanocarrier, which is not covered by the claims.

Biodegradable poly(D,L-lactide-co-glycolide) (PLGA) polymers with different molecular weights [Resomer RG 502 (MW 7.000-17.000 Da), Resomer RG 503 (MW 24.000-38.000 Da) and/or Resomer RG 504 (MW 38.000-54.000 Da)] and/or same crystallinity were selected to produce nanoparticles. These PLGA were modified to increase circulation time by using hydroxyl ended PEG (HO-PEG 3000) and methoxy ended PEG (Me-PEG 2000) as a monomer, synthesized by the ring-opening polymerization method (Vila et al). In order to couple the peptide on the PEGylated PLGA (MPEG-PLGA RG502, PEG-PLGA RG503, PEG-PLGA RG504) structures, maleic anhydride (MA) was grafted onto the backbone of the PEGylated products, followed by an amination step to obtain aminated maleic anhydride grafted PEGylated PLGAs (A-MA-PEG-PLGAs).

Blank nanoparticles, Teijin respectively Fluorescein isothiocyanate-bovine serum albumin (FITC-BSA) loaded-nanoparticles have been prepared by double emulsion solvent evaporation technique (Lemoine and Preat, 1998) using A-MA-PEG-PLGAs.

Finally, the 14 amino acids peptide, $NH_2$-VHPKQHRGGSKGCC-COOH (VC-14, which is a ligand for VCAM-1, was coupled to the surface of A-MA-PEG-PLGA nanoparticles using preferably 1-ethyl-3-(3-dimethylaminopropyl) carbodiimide (EDAC) as a coupling agent to prepare targeted PLGA.

The morphology of the modified PLGA nanoparticles have been analysed by scanning electron microscopy (SEM). Particle size was determined by Photon Correlation Spectroscopy and encapsulation efficiency of the particles has been determined.

The encapsulation efficiency of FITC-BSA into these nanoparticles was between 67.2%-82.1%, while Teijin was encapsulated between 19,6 % and 92 %.

According to these results, only modified medium and high molecular weight PLGAs were found to be suitable because of best encapsulation efficacy obtained with these nanoparticles.

The biocompatibility of PNP was established by an *in vitro* cytotoxicity test using cultured human vascular endothelial cells (HUVEC). The cytotoxicity of the particles was assessed by determining the viability of the cells after incubation with different concentrations of targeted PLGA nanoparticles by MTT assay.

Binding studies of the novel Nanocarriers

To study *in vitro* the binding of both types of Nanocarriers (TSL and the not claimed PNPs) to the target they were fluorescently labeled with Rhodamin-PE or FITC-BSA (70% loading efficiency), respectively. The specific binding of VCAM-1 directed FITC-labeled nanocarriers to cultured activated endothelial cells (human EAhy.926 line or mouse lung EC) was assessed under static and flow conditions by fluorescence microscopy and flow cytometry.

To investigate *in vivo* the localization of fluorescently labeled targeted nanoparticles, the experiments were performed using an atherosclerotic mouse model, the ApoE -/- mice and mice injected with MC-38iRFP tumor cells. Analysis was done with the help of the IVIS imaging system. Increased localization of VCAM-1 targeted Nanocarriers in the aortas of ApoE-/- was detected when compared with aortas from control C57BL mice. Also, the targeted nanoparticles accumulated in the lungs of mice injected with MC-38iRFP tumor cells.

Encapsulation of CA or CRA

The targeted nanocarriers (TSL and the not claimed PNPs) described in the present invention can be used for the delivery of chemokine antagonists (CA) or chemokine receptor antagonists (CRA) to the activated endothelium.

A number of commercially available compounds interfering with chemokines (CCL2, CCL5 and CX3CL1), or chemokine receptors (CCR1, CCR2, CCR5 and CX3CR1) have been selected and tested for incorporation into the targeted nanocarriers. Since these compounds differ in hydrophilic properties or amphiphilicity, thus displaying differences in loading and release characteristics, the nanocarriers have been tested for each loaded compound individually.

The CCR2 antagonist Teijin1: (*N*-[2-[[(3R)-1- [(4 -chlorophenyl) methyl]- 3- pyrrolidinyl ] amino]- 2- oxoethyl] -3 -(trifluoromethyl)benzamide hydrochloride) or the CCR5 antagonizing peptide D-ASTTTNYT (DAPTA; D-Ala-Peptide T-Amide) or a peptide sequence containing this sequence were selected for encapsulation into the target-sensitive Liposomes respectively the not claimed polymeric nanoparticles.

The incorporation rate of the CCR2-Antagonist Teijin or the CCR5 Antagonist DAPTA and their release in the culture medium after incubation with target EC was evaluated by HPLC.

The encapsulation efficiency of Teijin was about 75 $\mu$g Teijin/$\mu$mol liposomes as determined by HPLC. DAPTA was incorporated as well, but it was not possible to quantify the amount because of interference with the peptide ligand.

Teijin compound was also effectively loaded into the medium-, and high molecular weight targeted PLGA particles (not claimed) up to 90%. *In vitro* release studies showed that ~35% and ~10% of Teijin compound was released from medium molecular weight targeted PLGA and high molecular weight targeted PLGA nanoparticles in the first week, respectively, then the release rate slowed down and complete amount of Tejin was released after one month (medium molecular weight PLGA) and after more than 2 months from high molecular weight targeted PLGA.

Detection of the therapeutic effect of targeted nanocarriers: *in vitro* studies

The therapeutic effect of the targeted nanocarriers (TSL and not claimed PNPs) loaded with chemokine antagonists (CA) or chemokine receptor antagonists (CRA) as claimed in the present invention was investigated *in vitro* by measuring the decrease of monocyte or tumour cell adhesion and migration.

Activated endothelial cells in the presence or absence of CA/CRA-nanocarriers were incubated with fluorescently labelled monocytes. After thorough washing (to remove non-attached monocytes) the cultured cells (EC and attached monocytes) were lysed and the fluorescence was measured using a plate reader GENios, Tecan.

The monocyte transmigration was assessed using the double chambers assay.

The monocyte adhesion was investigated under dynamic flow conditions using the plate flow chamber assay. The adhesion or selectin-mediated rolling of monocytes to EC (cultured on microscopic slides) has been evaluated by a specific imaging system and quantified with respect to cell number and rolling velocity by specific imaging software. These studies were compared to Nanocarrier-pre-targeted EC or to the binding of monocytes in the presence of the targeted Nanocarriers. A clear impact of free Teijin or Teijin encapsulated in NC on the transmigration of either monocytes (U937) or melanoma cells in presence of monocytes were evident.

To facilitate metastasis in distant organs, tumor cells (TCs) need a favourable microenvironment induced by receptor-mediated interactions with platelets, leukocytes, fibroblasts, and EC. The interference with this complex communication of targeted CA/CRA-Nanocarriers was simulated by microscopic or flow cytometry studies and analyzed by detecting changes in cell-cell interaction. Two mouse carcinoma cell lines (breast carcinoma 4T1 and colon carcinoma MC-38 cells) have been used which are characterized with respect to adhesion, expression of chemokines and chemokine receptors and were found to efficiently metastasize in C57Bl6 mouse model. The adhesion process was assessed by dynamic microscopy. The tumor cells were pre-incubated with platelets, and/or leukocyte subpopulations before adhesion studies. To detect the impact of targeted CA/CRA-nanocarriers, the effects of pre-targeting the microvascular EC with nanocarriers on dynamic cell binding have been monitored. The changes induced by the released CA or CRA in cell adhesion characteristics have been validated by selective blocking the relevant adhesion receptors with antibodies or siRNA. Furthermore, the interactions of targeted nanocarriers to other cells (e.g. leukocytes, tumor cells) have also been analyzed by flow cytometry using fluorescently labelled nanocarriers. The efficacy of chemokine inhibition by targeted CA/CRA-nanocarriers on cell recruitment (monocytes, tumor cells) and endothelial transmigration have been assessed by classical transmigration assay in Boyden chambers.

<u>Detection of the therapeutic effect of targeted nanocarriers: *in vivo* studies</u>

Selected novel CA/CRA-nanocarriers (TSL and not claimed PLGA) were preclinical characterized in a comprehensive way including: determination of maximal tolerated dose of the nanocarriers; determination of side effects (body weight change, haematology, pathology); therapeutic efficacy; pharmacokinetic and pharmacodistribution, in the presence and absence of CA/CRA-nanocarriers in xenograft (human MT-3 breast cancer), syngeneic models (mouse breast cancer 4T1); and in PdT xenografts to define optimal dose and treatment schedule.

For example the Teijin loaded-PLGA nanoparticles, which are not covered by the claims were tested in the mouse for general toxicity and to determine the maximum tolerated dose (MTD) for therapeutic application. Nanoparticles were injected *i.v.* in concentration ranging between 2-250 mg/kg. It was found that the MTD was 125 mg/kg, which was tolerated well without any signs of side effects.

For preclinical *in vivo* testing of the novel nanocarriers, animal models of atherosclerosis (ApoE-deficient mice) and metastasis (mice with metastatic seeding of tumour cells or metastasis of tumour cells with chemokine knock-down, and patient-derived tumour models) have been employed.

Human tumors (cells or pieces) including seven different tumor entities (lung, colon, mammary carcinoma, pancreatic carcinoma, head and neck, melanoma and sarcoma) were successfully transplanted into the mouse models. Transplantation was modified according to different parameters (cell number, injection site, mouse strain, time) to evaluate their impact on metastasis development. These different tumor models from 6 tumors were used for testing the potential of the targeted CRA-loaded nanocarrier to inhibit metastasis *in vivo.*

Mice have been treated with CA/CRA-nanocarriers (TSL and not claimed PLGA) subsequent to the tumour cell injection (MC-38GFP, MT-3 or 4T1) and metastasis have been evaluated after four weeks for chemokine/chemokine receptor blockage and subsequent leukocyte recruitment. The distribution of circulating tumour cells and the targeted nanocarriers (not claimed nanoparticles/liposomes; all fluorescently labelled) has been analyzed by confocal laser scanning microscopy. The composition of tumour emboli around metastatic, GFP labelled tumour cells have been quantified using different leukocyte markers (e.g. Ly6C, Ly6G, F4/80).

To test the efficacy of CA/CRA-nanocarriers on cancer progression in mice primary tumor models (Lewis lung, 4T1) were used that spontaneously metastasize to the lungs. Mice with the growing primary tumour were treated with CA/CRA-nanocarriers at appropriate time points. Blood samples were analyzed for chemokines (at protein and transcription levels) and lungs were evaluated for the metastasis and recruitment of leukocytes. A panel of patient derived tumour (PdT) material from surgery sections focussing on tumours with metastatic potential has been established. The PdT was characterised for the relevant chemokine receptor expression at the gene and protein level by PCR and ELISA assays. Changes in chemokine receptor expression as a consequence of drug treatment were assessed similarly during the experiment, as well as the chemokine release. The novel nanocarriers have been tested in different PdT. Tumour volume inhibition, formation of metastases, side effects have been characterised after treatment and compared with controls and provided good results.

Assessment of the effect of targeted nanocarrier on adhesion of monocytes to explanted aortas from normal or ApoE-deficient mice have been done by *ex vivo* adhesion assay.

The lipid content of the atherosclerotic lesions in ApoE-deficient mice untreated or treated with CA/CRA-nanocarriers have been assessed microscopically after "en-face" Oil Red O staining of whole aorta. The atherosclerotic plaques size has been quantified employing AxioVision 4.8 Software (Carl Zeiss MicroImaging, Inc.).

Further the modulation of pro-inflammatory (IFN-$\gamma$ and IL-12) and anti-inflammatory (TGF-$\beta$, IL-4 and IL-10) cytokines secretion in the sera of ApoE-deficient mice untreated or treated with CA/CRA-nanocarriers was investigated.

The concept of targeted drug delivery using the novel nanocarriers provided by the present invention is an appealing therapeutic strategy because of its advantages with respect to the prior art. Among these advantages, the two types of nanocarriers of the present invention have the ability to target drugs specific and to restricted locations within the body in an efficient way, without being rapidly eliminated. The invention provides a carrier system which delivers effective concentrations of drugs to the diseased sites and reduces the drug concentrations at non-target sites. This results in a reduced, even absent, occurrence of side effects. The nanocarriers described in this invention have the potential to function as long-lasting vehicles for delivering selectively CA or CRA to activated endothelium.

**EMBODIMENTS**

The following examples are supposed to show how the invention can be carried out.

1. Preparation of target-sensitive liposomes (TSL) with encapsulated chemokine receptor antagonist (CRA)

Sterically stabilized target sensitive liposomes (TSL) as well as control non-sensitive liposomes (CL) were prepared by the lipid film hydration method in combination with extrusion and peptide conjugation as described previously [Paternostre M. et al., R. Prasad (ed.), Manual on Membrane Lipids, Springer-Verlag, Berlin, 1996, pp. 218Y226.]

To prepare TSL DOPE, DOPA and Mal-PEG-DSPE were solved in chloroform and mixed in different molar ratios (for details see Table 1). For the preparation of the control liposomes (CL) solutions of POPC, Chol, Mal-PEG-DSPE and PEG-DSPE were mixed in a molar ratio of 60:35:2:3.

In some cases, 1 mol % Rhodamine-PE was added as an ethanol solution subsequently before lipid film preparation, in order to fluorescently label the phospholipid bilayers of liposomes.

The organic solvent is evaporated by rotary evaporation under reduced pressure until a thin lipid film is obtained. The lipid film is dried and finally hydrated with phosphate buffered saline (PBS) to obtain final lipid concentration of 10 $\mu$mol/ml.

To obtain multilamellar vesicles (MLV), the lipid mixture was subjected to five cycles of freezing/de-freezing using liquid nitrogenous.

Small unilamellar vesicles were produced by extrusion of the MLV for 10 times through a 100 nm polycarbonate membrane using a Mini-Extruder (Avanti Polar Lipids, Alabaster, AL/USA).

To obtain CCR2- or CCR5 antagonist loaded TSL, multilamellar vesicles were prepared by lipid film hydration in combination with freeze/de-freezing and extrusion as described above.

Liposomes were obtained by hydration of the phospholipid film with a solution of Teijin or DAPTA. Unincorporated antagonist was removed by gel filtration using a Sephadex G-50 column and as an eluting solution the coupling buffer consisting of 10 mM $Na_2HPO_4$, 10 mM $NaH_2PO_4$, 2mM EDTA and 30 mM NaCl, pH:6.7.

Encapsulation efficiency of Teijin was about 75 $\mu$g Teijin/$\mu$mol liposomes as determined by HPLC (using UHPLC Agilent 1290 Infinity system with a column Zorbax SB C18 2.1x100 mm, 1.8 $\mu$m).

For content release studies, the liposomes were obtained by hydration of the phospholipid film with a solution of 100 mM calcein in 0.4 M NaOH (a fluorophore with self-quenching properties at high concentration). Unincorporated calcein was removed by gel filtration using a Sephadex G-50 column and as an eluting solution the coupling buffer consisting of 10 mM $Na_2HPO_4$, 10 mM $NaH_2PO_4$, 2mM EDTA and 30 mM NaCl, pH:6.7. Composition of control liposomes CL1 was: POPC - Chol - Mal-PEG-DSPE, molar ratio 60:35:2:3

1.1 Ligand coupling to the surface of target sensitive liposomes

A linear peptide affinity ligand, **VHPKQHR**, homologous to very late antigen-4, a known ligand for VCAM-1 is used to detect VCAM-1 in mouse atherosclerotic plaques and in human carotid arteries with atherosclerotic plaques (Nahrendorf et al, 2006).

The peptide with high affinity for EC adhesion molecule (VCAM-1) has been coupled to the distal end of a phospholipid derivate of PEG inserted into liposomes (targeted TSL).

A thioether bound between thiol (from peptide that was synthesized with a terminal cysteine) and maleimide-functionalized liposomes was used for coupling a peptide with high affinity for VCAM-1 to the liposome surface.

Before coupling, the peptide was reduced using tris (2-carboxyethyl) phosphine (TCEP) for 2 hours at room temperature in order to break the disulfide bond among the peptides and activate the sulfhydryl group. In order to remove the excess of TCEP, a dialysis (using cellulose ester membrane with a cut off of 500-1000 Da) against coupling buffer was performed overnight at 40C. Then, an aliquot of the solution of the VCAM-1 recognizing peptide in coupling buffer was added at a concentration of 10 $\mu$g peptide/$\mu$mol total lipid to maleimide modified PEG-DSPE containing liposomes and incubated overnight at room temperature under shaking. To saturate un-reacted maleimide, 1 mM L-cystein was added for 30 minutes at room temperature and the unbound peptide was removed employing centrifugal filter columns with a cut off of 100 kDa. Control liposomes (without peptide coupled) were prepared from the same lipid mixture and the reactive maleimide groups were blocked with L-cystein.

The amount of peptide coupled at the surface of liposomes was quantified by HPLC using a UHPLC-Agilent 1290 Infinity with a column: Zorbax SB C18 2.1x100 mm, 1.8 $\mu$m using a gradient of 0.1 % TFA in water (A) and 0.1 % TFA in acetonitrile (B) (from 1% B until 26% B in 10 minutes). The coupling efficiency was expressed as $\mu$g peptide/$\mu$mol phospholipid. Under these conditions, the peptide was quantitatively conjugated to the liposomes resulting in coupling efficiency of 10 $\mu$g VCM-1 directed peptide/$\mu$mol phospholipid.

1.2 Liposomal characteristics

Liposomes prepared as described above were characterized regarding physical properties (size, stability) and functionality (binding, drug release,cytotoxicity)

*Size:* The size distribution and the average diameter of the resulting liposomes was determined by photon correlation spectroscopy using Nicomp submicron particle analyzer model 380 (Nicomp Inst Corp, Santa Barbara, CA) employing multi-modal distribution. The vesicle suspensions were diluted into freshly 0.22 $\mu$m-filtrated PBS in order to reach a count rate of 250 kHz to 350 kHz. The instrument parameters were set as follows: automatic choice of channel width, vesicle mode, number weighting and automatic change from Gaussian distribution mode to multimodal mode if the value of Chi-squared exceeded 3.00.

All liposomes were prepared preferable in a size of 100 - 200 nm and had a unimodal size distribution, as shown by the polydispersity index, which was always smaller than 0.2. There were no significant differences between peptide-coupled and non-targeted TSL with respect to size characteristics immediately after preparation or during storing periods.

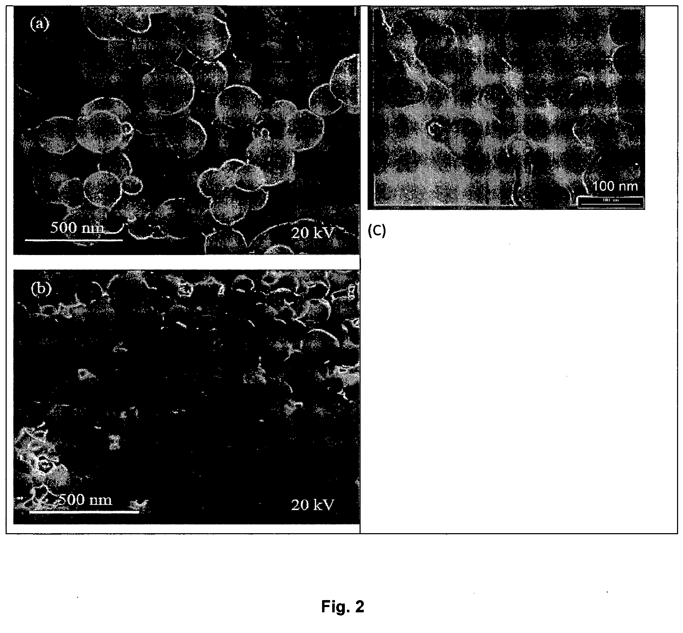

*Microscopy:* The liposomes were visualized by transmission electron microscopy (TEM) upon negative staining with 1% phosphotungstic acid using an transmission electron microscope Philips EM 410 (figure 2).

*Storage stability:* To test the storage stability (at 4°C), the leakage of the liposomes-entrapped calcein was quantified. Calcein was encapsulated into the liposomes at a concentration of 100 mM, when its fluorescence is self-quenched and has thus a low value. The calcein release after storage at 4°C for different periods of time (up to 50 days) was determined using in 96 well black plates by measuring the increase in fluorescence intensity with excitation and emission wavelengths of 490 nm and 520 nm with a spectrofluorimeter plate reader TECAN. The percentage of calcein released was calculated according to the formula introduced by Ho et al, 1986:

$$\% \text{ calcein release} = (F-F_0)/(F_{Tx}-F_0)x100$$

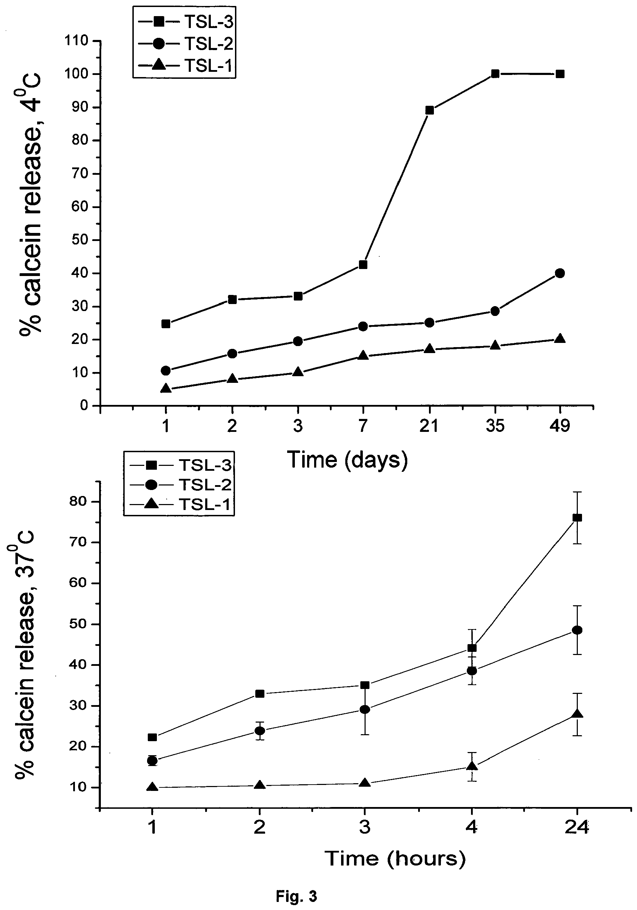

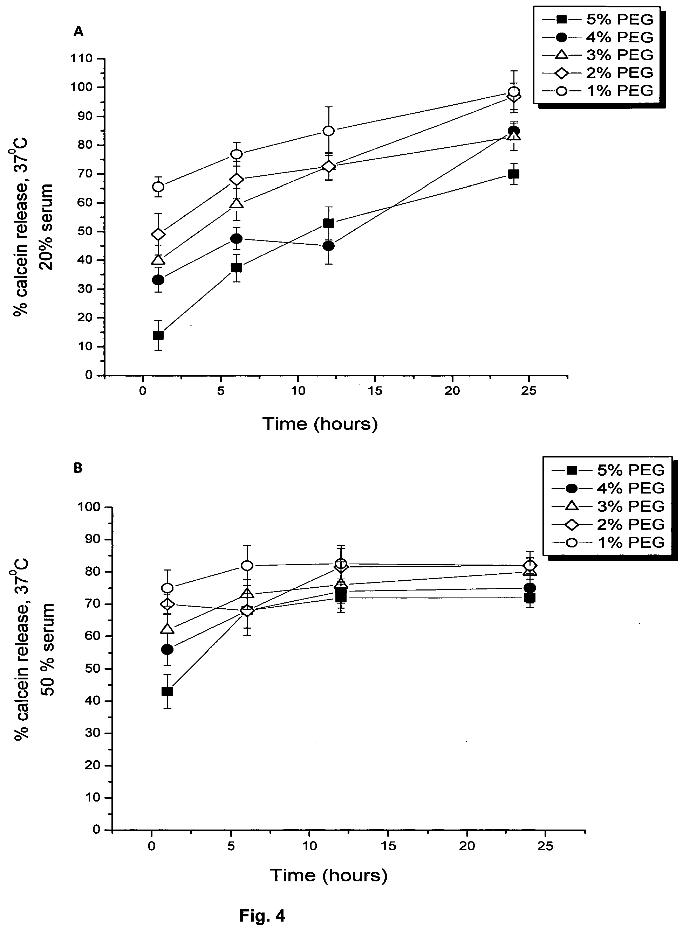

where $F_0$ represents the calcein fluorescence immediately after liposome preparation, F is the calcein fluorescence at a certain time point and $F_{Tx}$ is the total fluorescence after adding 1% Triton X-100. Figure 3 shows the results for the calcein release at 4°C and 37°C, respectively. The best results regarding the stability could be obtained for Liposomes with a DOPA content of 35%. Using lipid compositions with this DOPA concentration, no noticeable increase of calcein released within 50 days was detected. *Serum stability of liposomes:* Liposomes with different content of PEG-lipids, encapsulated calcein at a self-quenching concentration were added to wells of a 96-well black plate containing 20 % or 50 % serum in PBS and the incubation was performed at 37°C for 24 hours using a PHERAstar reader BMG Labtech equipped with a temperature controlled chamber. The increase in calcein fluorescence over time was measured and analysed using Mars software. The calcein release is summarized in figure 4. was determined using formula [1] where $F_0$ and F represent the calcein fluorescence before and after incubation with serum and $F_{Tx}$ is the total fluorescence after adding 1% Triton X-100.

2. Preparation and characterization of polymeric nanoparticles (PLGAs)

(Reference example)

The Embodiments disclosed in the subsequent Sections 2.1-2.7 adress the unclaimed type of nanocarrier composed of polymeric nanoparticles, which is not covered by the claims.

2.1. Synthesis of PEG-PLGA copolymers to obtain nanocarrier with long circulating characteristics (not claimed)

PLGA polymers with different molecular weights [Resomer RG 502 (MW 7.000-17.000 Da), Resomer RG 503 (MW 24.000-38.000 Da) and Resomer RG 504 (MW 38.000-54.000 Da)] and same crystallinity (the ratio of lactic acid and glycolic acid of all PLGA polymers were 50:50) have chosen to produce targeted PLGA nanoparticles. All Resomers were purchased from Evonik Industries Röhm GmbH, Germany. Resomer RGPd 501155, a commercial PEGylated PLGA product (with PEG 5.000, purchased from Evonik Industries, Germany) was used as control.

PEG-PLGA copolymers were synthesised based on hydroxyl ended PEG (HO-PEG 3000, Sigma-Aldrich, USA) and methoxy ended PEG (Me-PEG 2000, Sigma-Aldrich, USA) as a monomer, dibutyl tin dilaurat (0,2%, w/w, of PLGA) as a catalyst according to ring-opening polymerization method (Vila et al., 2004). Briefly, 10 g PLGA and 2 g Me-PEG or HO-PEG were dissolved in 150 ml toluene. The solution was transferred to the 500 mL five-necked flask equipped with mechanical stirrer, nitrogen inlet and outlet, water-cooled condenser in an oil bath. Then, 20 mg dibutyl tin dilaurat was added to the solution and the solution was heated 114°C for 8 h. At the end of the reaction, the solvent was evaporated by the rotary evaporator and the residue was dissolved in 200 mL dichloromethane. Then, the solution was added to the distilled water which was stirred vigorously at 60°C and dichloromethane was evaporated from the emulsion and finally the PEG-PLGA copolymer was separated by decantation. It was dried in vacuum at room temperature and stored at 4°C.

2.2. Synthesis of maleic anhydride grafted PEGylated PLGA copolymers (MA-PEG-PLGAs) (not claimed)

In order to couple the peptide on the PEGylated PLGA (MPEG-PLGA RG502, PEG-PLGA RG503, PEG-PLGA RG504) structures, firstly, maleic anhydride (MA) which is biocompatible and provides highly reactive groups for further chemical modification, was grafted onto the backbone of the PEGylated products. Grafting reaction was carried out according to the melt free radical grafting reaction by using benzoyl peroxide (BPO) as the initiator (Pan et al., 2005; Niu et al., 2008; Luo et al., 2008). MA (99%) and BPO (for synthesis) were purchased from Merck AG (Germany) and Sigma-Aldrich (Germany), respectively.

In the grafting reaction, the ratio (w/w) of PEGylated copolymers (PEG-PLGA RG502, PEG-PLGA RG503, PEG-PLGA RG504 and RGP d501155) to MA has been selected as 10:1 and BPO was used as 5% of MA weight. The mixture containing 200 mg PEGylated polymer, 20 mg MA and 1 mg BPO were put into the 5 ml round-bottom glass flask and dried under vacuum at room temperature for 24 h. Then, oxygen-free nitrogen gas was purged in the flask and immediately and tightly sealed with glass stopper. Afterwards, flask were heated to $100 \pm 1$°C under vacuum and allowed at the same temperature for 24 h. After cooling the flask to room temperature, the product was extracted with purified water at 50°C for 15 min to remove the unreacted maleic anhydride in the polymer. Purified product was dried under vacuum at room temperature for 24 h.

2.3. Amination of anhydride groups of MA-PEG-PLGAs (A-MA-PEG-PLGAs) (not claimed)

Anhydride groups in MA-PEG-PLGAs were aminated by a diamine compound such as hexamethylenediamine (HMDA) before the coupling of the peptide. 150 mg of MA grafted PEGylated polymer and HMDA were dissolved in 750 $\mu$l and 75 $\mu$l of tetrahydrofuran, respectively. Thereafter, the HMDA solution was dropped into the MA grafted PEGylated polymer solution with stirring at a temperature below 20°C for 40 min. After reaction, the amination MA grafted PEGylated polymer was dissolved in the tetrahydrofuran and subsequently precipitated in distilled water at pH value 7-8. Then, the precipitated product was filtered and washed with distilled water at least three times. Finally, it was dried under vacuum at room temperature to constant weight for at least 48 h. Aminated products (A-MA-PEG-PLGAs) have been used for the fabrication of nanoparticles.

2.4. Preparation of aminated maleic anhydride grafted PEGylated PLGA (A-MA-PEG-PLGA) nanoparticles (not claimed)

Blank nanoparticles, Teijin compound 1 and Fluorescein isothiocyanate-bovine serum albumin (FITC-BSA) loaded-nanoparticles have been prepared by double emulsion solvent evaporation technique (Lemoine and Preat, 1998) using aminated maleic anhydride grafted PEGylated PLGAs (A-MA-PEG-PLGAs). An inner aqueous phase ($W_1$) containing polyvinyl alcohol 2.8% (w/v) was emulsified in dichloromethane (DCM) containing 2% (w/v) copolymer ($O$) by homogeniser (CAT X620, Germany) at 9.500 rpm for 75 sec in an ice bath to form the primary emulsion ($W_1/O$). In order to prepare the drug-loaded nanoparticles, 2 mg Teijin compound 1 or 5 mg FITC-BSA was dissolved in $W_1$. The primary emulsion was then re-emulsified in an outer aqueous phase ($W_2$) containing PVA 0.4% (w/v) by homogeniser (CAT X620) at 9.500 rpm for 105 sec to form the double emulsion ($W_1/O/W_2$). Then, the double emulsion obtained was poured into the dilution solution containing 0.1% PVA and homogenised at 9.500 rpm for another 105 sec. The emulsion was stirred for 30 min on a magnetic stirrer at room temperature and then organic solvent was evaporated under vacuum of 50 mbar by rotary evaporator (Thermo Scientific, USA) for 15 min. Nanoparticles were separated by centrifugation and VCAM-1 specific peptide coupled on their surfaces.

2.5. Coupling of Vascular cell adhesion molecule-1 (VCAM-1) specific peptide onto the A-MA-PEG-PLGA nanoparticles (peptide- A-MA-PEG-PLGA) **(not claimed)**

Vascular cell adhesion molecule-1 (VCAM-1) is an immunoglobulin-like adhesion molecule over-expressed by activated endothelial cells covering atherosclerotic plaque and by endothelial cells found in a metastatic microenvironment (on tumour vessels). A Peptide with 14 amino acids, $NH_2$-VHPKQHRGGSKGCC-COOH (VC-14, GeneCust, Luxembourg), which is a ligand for VCAM-1, has been coupled to surface of A-MA-PEG-PLGA nanoparticles via a stable covalent amide bond by using 1-ethyl-3-(3-dimethylaminopropyl) carbodiimide (EDAC) as a coupling agent for targeting the nanoparticles to activated endothelial cells.

10 mg peptide was dissolved in 1 ml purified water. Then, 10 mg EDAC was added in the peptide solution and stirred for 3 h on Magnetic stirrer in the darkroom at 15°C to activate the carboxylic acid group of peptide. 100 mg A-MA-PEG-PLGA nanoparticles was dispersed in 10 ml purified water and then activated peptide solution was added in the nanoparticle dispersion while stirring at 300 rpm on magnetic stirrer for 12 h for reacting of amine groups of nanoparticles and the carboxylic acid groups of peptide. Then, excessive EDAC and unreacted peptide was removed by either dialysis the nanoparticles against distilled water for 4 h or centrifuging and washing the particles 3 times. Then, a cryoprotectant, trehalose, and a nonionic surfactant, Tween 20, were added in the peptide coupled nanoparticle dispersion and freeze-dried.

Properties of prepared targeted nanoparticles are summarized in Table 2.

### 2.6. Targeted PLGA nanoparticle characteristics (not claimed)

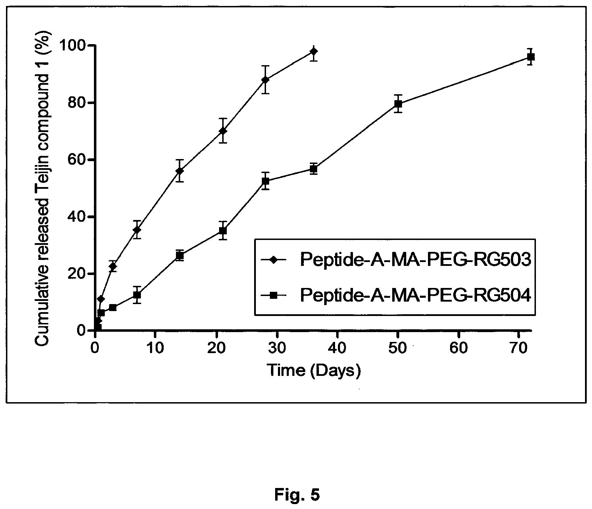

*Teijin compound 1 release from targeted PLGA nanoparticles: In vitro* release studies showed that ~35% and ~10% of Teijin compound was released from medium molecular weight PLGA and high molecular weight PLGA nanoparticles in the first week, respectively, later the release rate slowed down and 100% content was released after about one month (medium molecular weight PLGA) and after more than 2 months (high molecular weight PLGA , figure 5).

### 2.7. In vitro cytotoxicity Test of CRA-entrapped nanocarriers (not claimed)

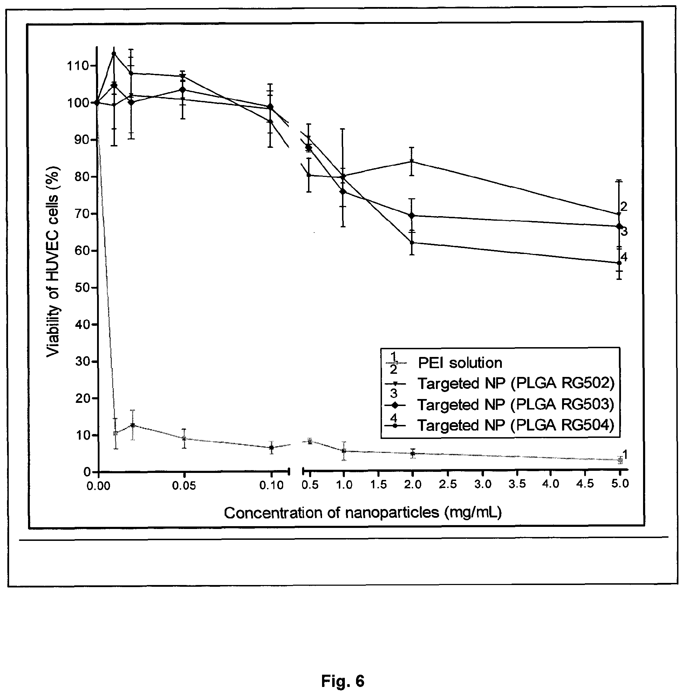

The cytotoxicity of the particles was assessed by determining the viability of HUVECs after incubation with different concentrations of targeted PLGA by MTT assay. At a concentration of 5 mg/ml, the cell viability was between 56-70% and a decrease in molecular weight of PLGA slightly reduced the toxicity of investigated nanoparticles (figure 6).

### 3. *In vitro* analysis of the interaction of target-sensitive liposomes with the activated endothelial cells

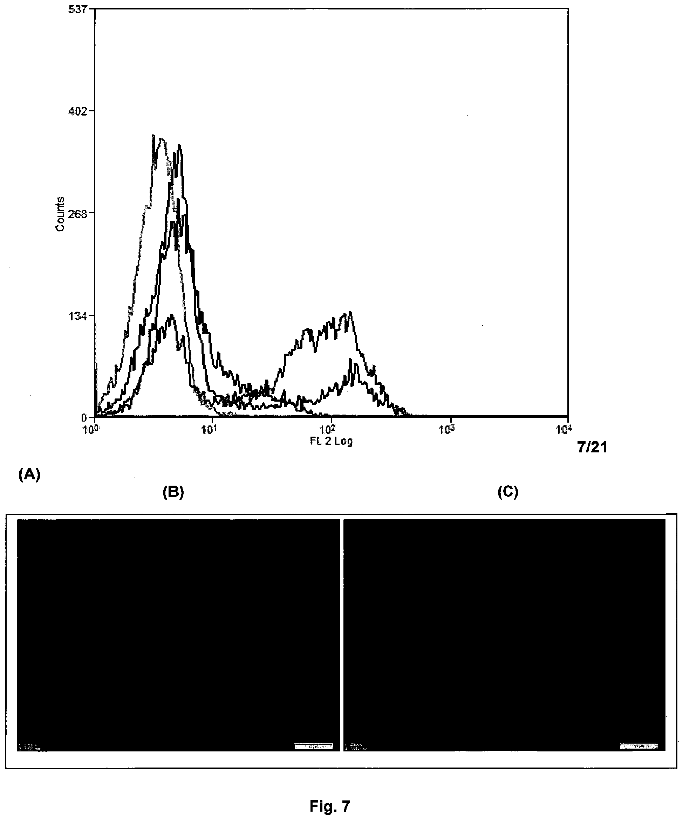

The activated or quiescent endothelial cells were slightly fixed in 1 % PFA and incubated for 1 h at RT and incubated with 0.25 $\mu$mol/ml Rhodamine-PE-labelled targeted or non-targeted TSL in 100 $\mu$l incubation solution containing and 1 mg/ml BSA in PBS. To remove unbound liposomes, the cell/liposome suspensions were washed 3 times with cold PBS and then analyzed by flow cytometry (Moflo Dako; figure 7A) and fluorescence microscopy (figure 7B and 7C). These studies demonstrate that non-targeted TSL do not bind or bind to a very low level to EC (<1%). Targeted TSL bind specifically to activated ECs; the binding was significantly higher comparing with the binding to non-activated EC (40 % versus 10%, respectively (figure 7A).

To assess the specific binding of VCAM-1 targeted liposomes to activated endothelial cells, a competitive binding assay was performed. The TSL equipped with the peptide with VCAM-1 affinity were pre-incubated for 30 minutes with an excess of soluble VCAM-1 (10 $\mu$g/ml) before incubation with endothelial cells. The EC monolayers were washed three times to remove unbound liposomes.

The specific binding of TSL to activated ECs (figure 7B) can be completely blocked by saturation of activated ECs with VCAM-1 recombinant protein prior to addition of the liposomes (figure 7C)

### 4. Marker release from liposomes after binding to the endothelial cells under static conditions

A prerequisite to enable a high activity at target site is the destabilization of the liposomes to release the encapsulated payload after binding to the target cells.

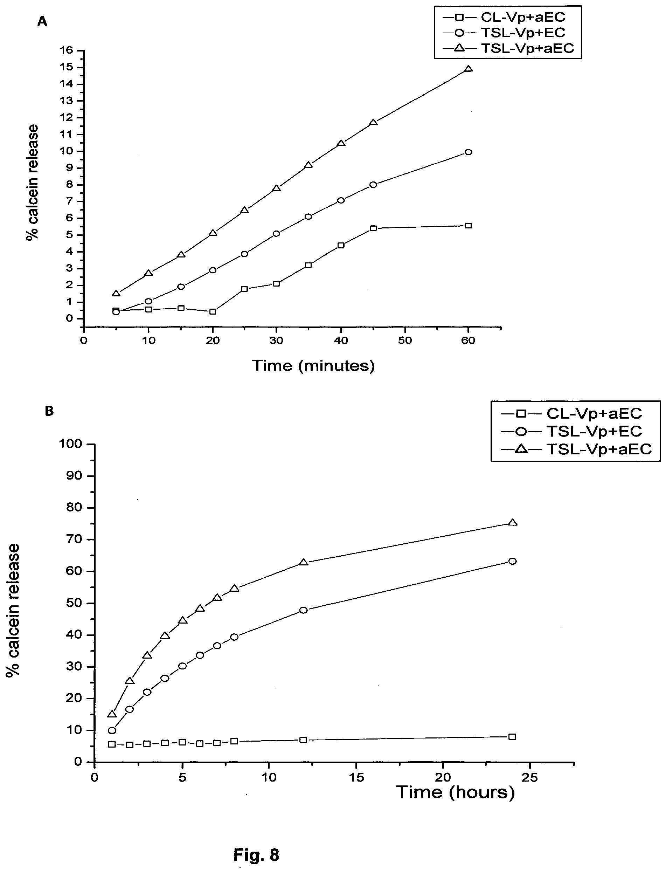

The kinetic of calcein (used as model compound) release from 10 $\mu$M TSL equipped with 5 mol % PEG incubated with quiescent or TNF-$\alpha$ activated EC fixed in 1% paraformaldehyde was followed for periods from 1 to 24 hours at 37°C by fluorescence measurements. The data, shown in Fig. 8, indicate that TSL, bound to activated ECs, released always the most amount of marker with values of 15% and 74% after 1 and 24 h, respectively. Control liposomes CL-1 released finally after 24 h of measurement only 6% of encapsulated marker (figure 8).

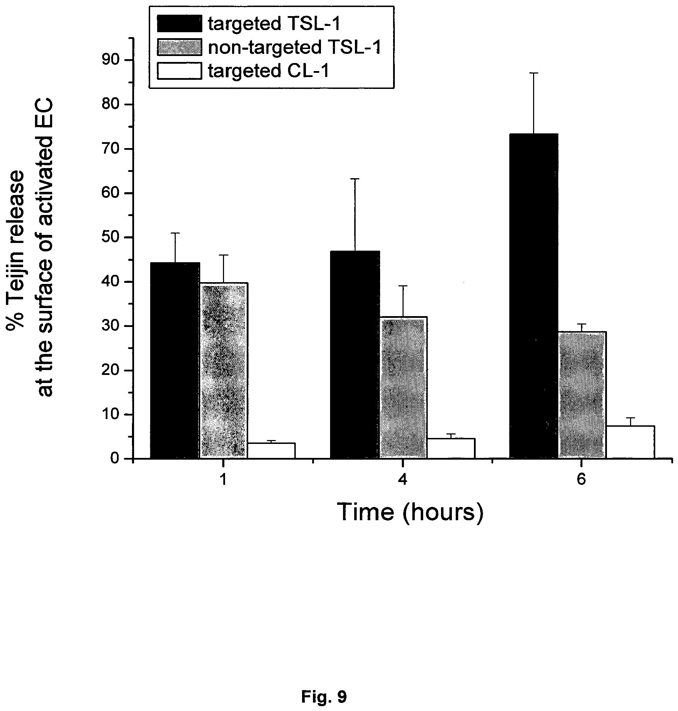

The results obtained with marker molecule calcein were confirmed also for liposomes containing the CCR-2 antagonist Teijin compound 1. Therefore, Teijin loaded targeted TSL-1 and CL-1 or in non-targeted TSL-1 (1 $\mu$mol/ml) were added to EC and after defined times, the media (1 ml) were collected and intact liposomes were separated from released Teijin using Millipore centrifugal filter units of 100K cutoff. Teijin was determined by HPLC and the release was calculated in relation to the start concentration of Teijin in liposomes before incubation. Again, the best release data were obtained with targeted TSL-1 with values of 43% after 1 hour and more than 78% after 6 hours, while best values released from non-targeted TSL-1 and targeted CL-1 were aprox. 30% and 7% after 6 hours, respectively (figure 9).

### 5. Content release from TSL after incubation with EC flow conditions

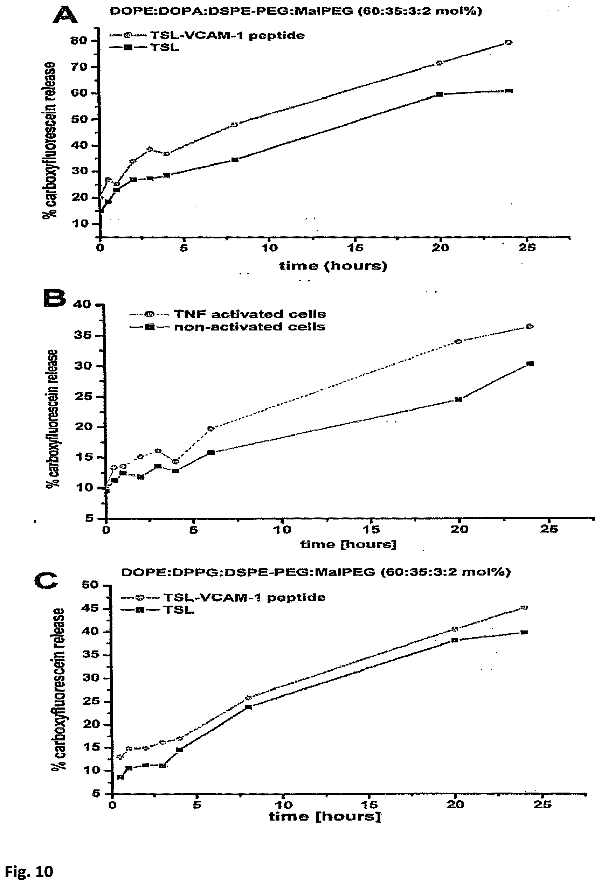

In flow chamber experiments, glass slides were covered with 0.2 $\mu$g VCAM-1 Fc chimera or with quiescent or TNFα-activated EC. Slides were incorporated into a parallel plate flow chamber, the flow chamber was fixed on an inverted microscope, and PBS (pH 7.4) flow medium was driven at a shear rate of about 200 s$^{-1}$. Different kinds of liposomes were added to a total volume of 2 mL and calcein release was determined at different time points within 24 h. For this, calcein-fluorescence of 50 μL aliquots was quantified with a POLARstar Galaxy plate reader (BMG Labtech). Data are shown in figure 10.

6. Inhibition of monocyte adhesion to immobilised EC by TSL in vitro

The effect of CCR2 antagonist,Teijin compound 1 on monocyte adhesion to activated endothelial cells was followed using ECs seeded in 24-well plates and activated for 24 hours with 10 ng/ml TNF-α. The activated ECs were preincubated for 1 hour with different concentrations of Teijin free or encapsulated into targeted or non-targeted TSL-1 or into targeted CL-1. After 1 hour, fluorescently labelled monocytes were added over the EC in the incubation media. The number of adhered monocytes was determined and the percent inhibition of monocyte adhesion to activated EC in the presence of free or liposome-encapsulated Teijin was calculated.

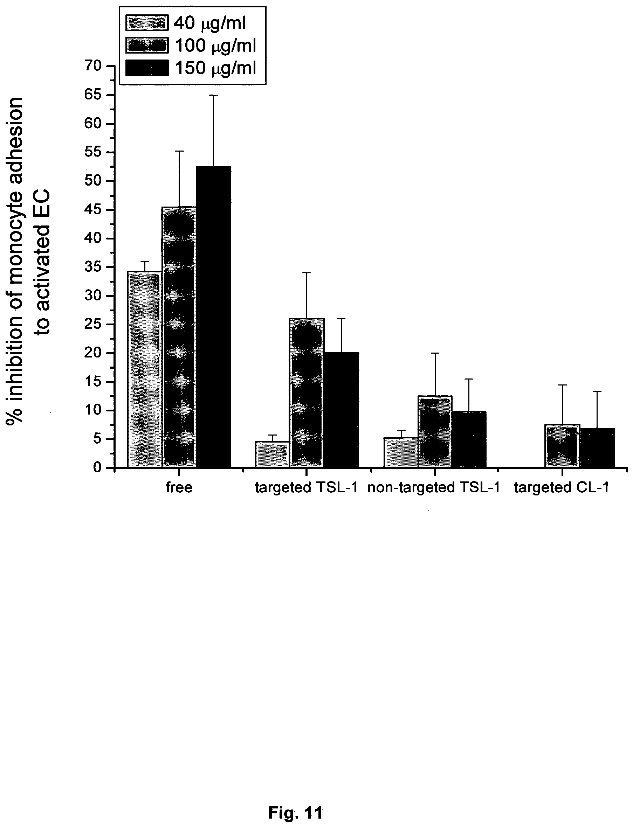

It could be demonstrated that adhesion of monocytes to activated, immobilized ECs can be inhibited by about 60% in the presence of Teijin loaded targeted TSL-1 in comparison to that obtained with the free Teijin (figure 11). The absolute inhibition was 28%, obtained with a concentration of 100 μg Teijin incorporated into targeted TSL-1/ml. A further increase of the concentration does not enhance the effect. The inhibition by non-targeted TSL and targeted CL-1 was significantly lower with values of 13% and 7%, respectively.

7. TSL destabilization after binding to VCAM-1 on the surface of activated endothelial cells

The liposomal disruption after binding to the EC surface has been simulated using a SAW biosensor system and atomic force microscopy.

7.1. SAW binding studies

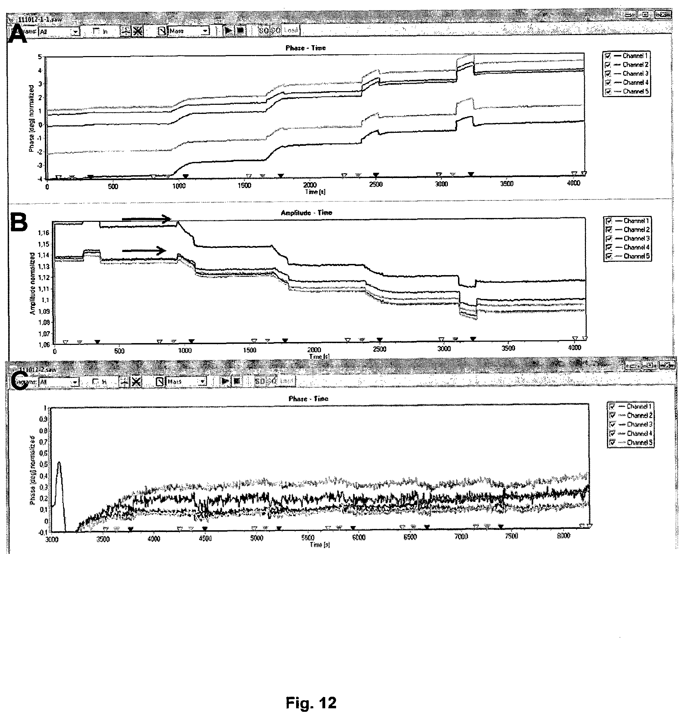

The liposomal binding onto a VCAM-1 covered sensor was investigated by the mass-sensitive technology of Surface Acoustic Wave sensors. It became clearly evident that VCAM-1 directed TSL bind to the target protein, but not the plain TSL without the peptide. Considering the change in oscillation amplitude as a parameter for surface viscoelasticity, an increase in amplitude after initial binding of the targeted TSL were evident, which indicates an intensive surface contact of the liposomes, most likely a flattening or fusion at the target surface (figure 12). This could not be achieved using peptide modified liposomes of conventional, not TSL lipid composition.

7.2. AFM studies

For topographic investigations the same gold sensors as for SAW-sensor measurements were used. The technique used was Scanning Polarization Force Microscopy (SPFM) that uses the electrostatic interactions between a charged tip and the sample to investigate the topography. The technique is a true non-contact method as the metallic tip is kept at 20nm from the sample. The gold surface of the sensors was covered with BSA or rhVCAM-1 Fc chimera that was immobilized ex situ via cyanurchlorid. The SPFM scans were performed in AC mode with 5V amplitude bias applied between the tip and sample at 3kHz. The surface of the sensors was investigated both in dry and hydrated conditions. A tissue paper was used to remove the excess water from the side of the sensor after hydration (the surface was never touched). The surfaces before and after hydration show no change in the surface topography. The microscope chamber had ~60% humidity so that the water film on the surface of the sensor would not evaporate too quickly.

After the surface has been initially scanned a few drops of the liposomes solution was added and left for 20 minutes to adhere to the surface. The excess solution was removed by absorption with a tissue paper from the side without touching the investigated surface.

Figure 12 shows SPFM scans on the surface after the addition of VCAM-1 targeted TSL liposomes. The presence of features 30-50nm in height and 150-250nm in diameter confirms the adhesion of the liposomes to the VCAM-1 functionalized surface.

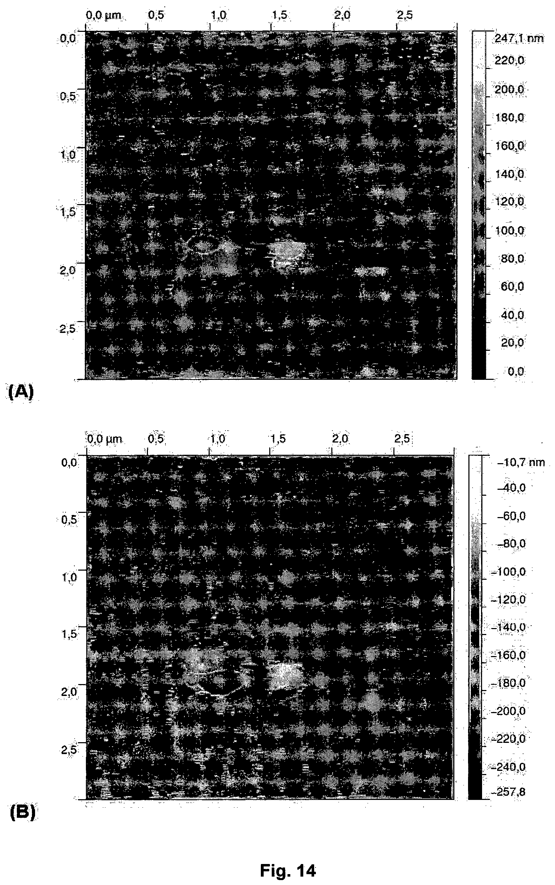

The volume of the liposomes has been estimated from the topography images. The difference in height of the liposome compared to the surface height can be measured and integrated over the 110 nm high liposome and over the collapsed liposome. The red area in figure 14 marks the calculated volumes. The volume of the 110nm high liposome, presented in figure 14a), is 0.00177 μm$^3$, while the volume of the collapsed liposome in figure 14b) is 0.00179 μm$^3$. The volume of a liposome after collapsing is not expected to change as the same amount of liquid is present only rearranged as a droplet instead of a bilayer micelle. The estimated volume of the liposomes is very similar indicating that the image presents a collapsed and a non-collapsed liposome.

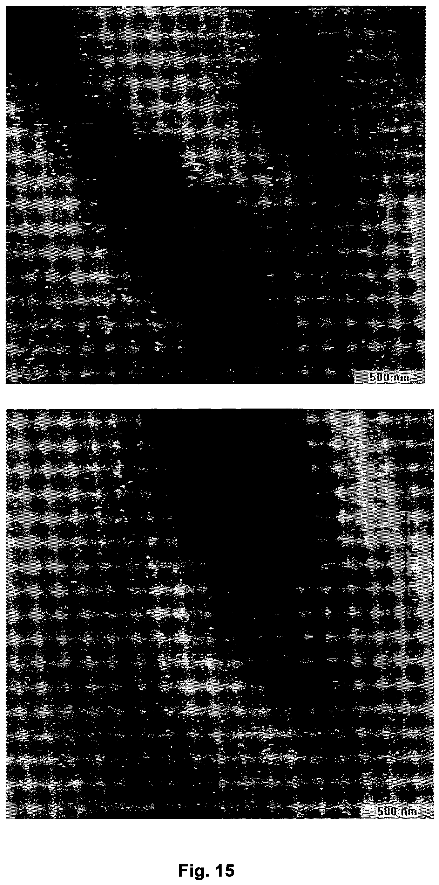

Similar samples of non-sensitive liposomes deposited on the VCAM-1 functionalized surface have been investigated by SPFM. These samples have not shown the adhesion of the liposomes to the substrate. Another control experiment using BSA covered surfaces shows that neither control liposomes nor the target-sensitive liposomes adhere to the BSA covered surface as shown in Figure 15.

### 8. Inhibition of monocyte transmigration through activated EC monolaver by TSL in vitro

The efficacy of chemokine receptor inhibition by Teijin-loaded targeted TSL-1 on monocytes transmigration through activated endothelial cells have been assessed by classical transmigration assay using Boyden chambers. The EC were seeded on the insert's filter and activated for 24 hours with 10 ng/ml TNF-$\alpha$. Then, activated EC were incubated with 100 $\mu$g/ml Teijin free or encapsulated into targeted or non-targeted TSL-1 or into targeted CL-1 in the presence of calcein-AM labelled monocytes. After 16 hours, the fluorescence in the lower chamber was measured and the percent of inhibition of monocyte transmigration was determined relative to the control (monocytes incubated with activated cells in the absence of free or liposome-encapsulated Teijin).

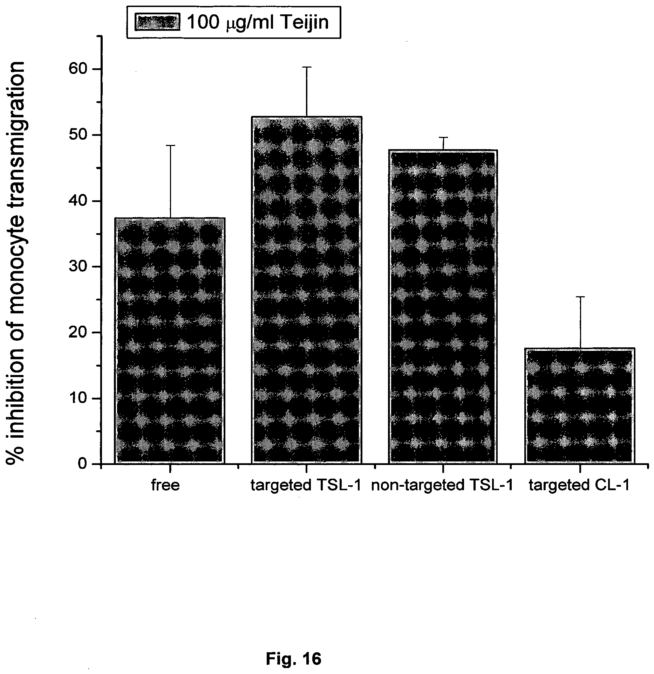

The results (Figure 16) demonstrate that Teijin-loaded TSL liposomes inhibited the transmigration of monocytes better than the free anatgonist or than control liposomes loaded with Teijin.

### 9. Assessment of the effect of Teiiin-encapsulated TSL-1 on atherosclerotic plaque development Apo-E-deficient mice

To follow the effect of Teijin-encapsulated TSL-1 on the development of atherosclerotic plaque, ApoE-deficient mice at 7 weeks of age were fed with high-fat, Western-type diet and were intravenously injected three times/week for six weeks with free Teijin (in 5 % mannitol) or with Teijin encapsulated into targeted or non-targeted TSL-1 (in PBS) or with PBS or 5% mannitol (controls). Twenty eight ApoE-deficient mice were divided into 5 experimental groups: (1) 6 animals received 50 $\mu$g free Teijin in 5 % mannitol (free Teijin group); (2) 6 animals injected with PBS containing 50 $\mu$g Teijin encapsulated into targeted TSL-1 (targeted TSL-1 group); (3) 6 animals injected with PBS containing 50 $\mu$g Teijin encapsulated into non-targeted TSL-1 (non-targeted TSL-1 group); (4) 6 animals injected with PBS (control group); (5) 4 animals injected with 5 % mannitol (control group).

At the end of the experiment, mice were anesthetized with ketamine /xylasine and the vasculature was washed by injection into the left ventricle of the heart of PBS buffer using a peristaltic pump (infusion rate 2.5 ml/min). The aortas were excised and were mounted *en face* and stained with Oil Red O. The total surface areas of the aortic arch, thoracic and abdominal aorta and the areas of *atherosclerotic lesions* were quantitated using AxioVision 4.8 Software (Carl Zeiss Microlmaging, Inc.). The atherosclerotic lesions areas were expressed as % of total aortic area.

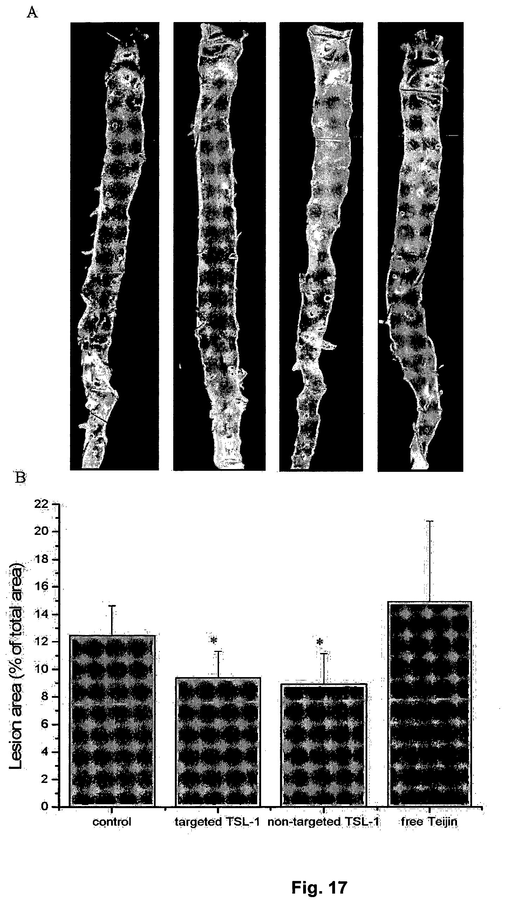

The results showed no difference between control groups injected with PBS or 5 % mannitol, therefore in the Figure 17B we represented as control group the median value obtained from all these animals. A significantly inhibition of atherosclerotic lesions area by approximately 25 % in targeted TSL-1 group in comparison with control was obtained (p=0.02). No difference in the lesions area was obtained between targeted TSL-1 group (9.37 $\pm$ 1.94 %) and non-targeted TSL-1 group (8.92 $\pm$ 2.23 %). The administration of free Teijin determined a slightly increase in atherosclerotic lesions (14.92 $\pm$ 5.86 %) at a level that was not significantly different in comparison with control group (12.47 $\pm$ 2.17 %) (figure 17).

### 10. Binding of liposomes to aortas of ApoE-deficient mice

To follow the specific binding of targeted liposomes to ApoE-deficient mice aorta, CL-1 were administrated in situ or in vivo to ApoE-deficient mice (at 14 weeks of age, after 6 weeks of high-fat, Western-type diet) or control, C57BL mice (kept on chow diet).

In situ experiments consisted of the following: mice were anesthetized with ketamine / xylasine, then the vasculature was washed by injection into the left ventricle of PBS buffer using a peristaltic pump (infusion rate 2.5 ml/min) followed by administration of fluorescently (Rhodamine-PE) labeled non-targeted or targeted CL-1 at a concentration of 1$\mu$mol/ml. Liposomes were left to stay for 20 minutes in the aorta by performing a ligature in the abdominal aorta. The ligature removal was followed by extensive washing vasculature for 10 minutes (2.5 ml/min.) to remove liposomes that are not specifically bound to vascular endothelium.

For in vivo administration, mice were anesthetized with a small dose of anesthetic ketamine / xylasine and fluorescently (Rhodamine-PE) labelled non-targeted or targeted CL-1 were intravenously injected. After one hour, the mice were anesthetized again and the vasculature was washed using a peristaltic pump (infusion rate 2.5 ml/min) by injection of PBS buffer into the left ventricle.

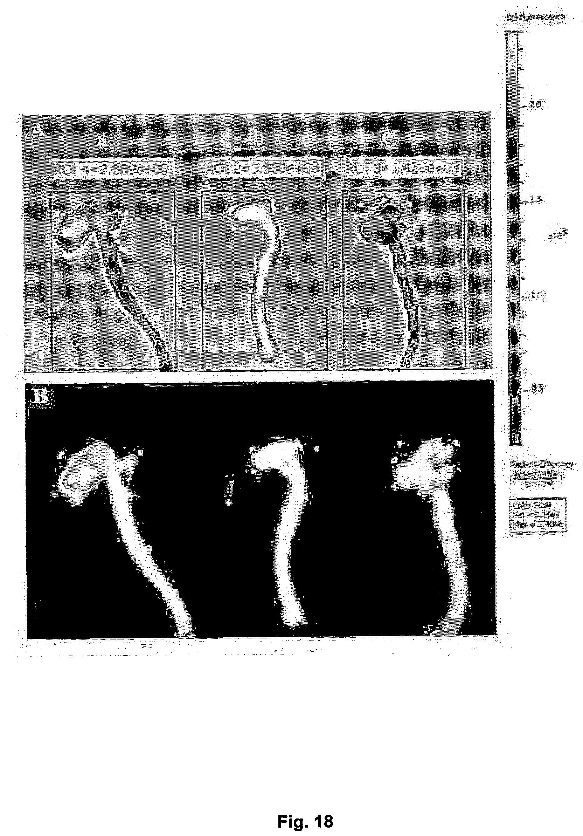

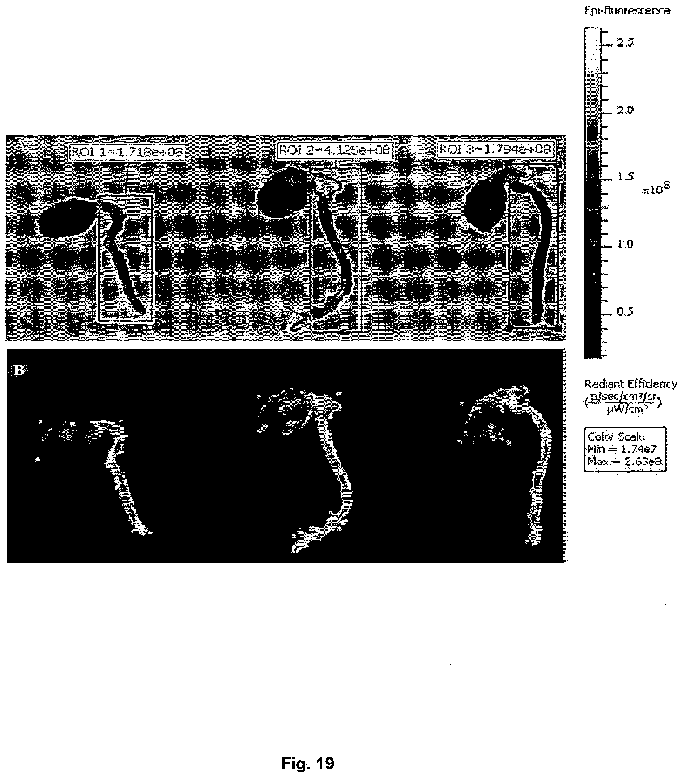

In both cases, the aortas were excised and the fluorescence was measured using an IVIS Imaging System 200 (Caliper Life Sciences) using $\lambda_{ex}$ = 535 nm and $\lambda_{em}$ = 620 nm (figures 18 and 19).

The results revealed a specific binding of targeted CL-1 in comparison with non-targeted CL-1 to the aorta of Apo E-/- mice and no binding to the aorta of control C57BL mice (figures 18 and 19).

## 11. Establishment of a metastatic patient derived tumor model the mouse for *in vivo* studies

Tumor pieces from primary lung carcinoma LU 9313M were transplanted s.c into the left flank of female NSG mice. At day 31 mice were sacrificed and tumors were isolated, separated into single cell suspension by treatment with a Gentle MACS.

A defined number of LU 9313 tumor cells (between $1\times10^4$ - $1\times10^6$, suspended in 200 $\mu$l puffer) were injected s.c. into the left flank of each of three female NSG mice. After different times (e.g. day 31) mice of indicated groups were sacrificed and liver and lung was isolated, weighed and the number of macroscopically visible metastases were counted.

Finally, tissues were shock frozen for PCR analysis to determine the quantity of human tumor cell in the mouse tissue as another parameter of metastasis.

Therefore, a 3 x 3 mm (approx. 20mg) piece of tissue is disrupted and homogenized in the provided Qiagen® ATL lysis buffer under addition of 20$\mu$l proteinase K. 50-100$\mu$l blood samples or $5\times10^6$ cells are lyzed and homogenized in the provided Qiagen® AL lysis buffer under addition of 20$\mu$l proteinase K. DNA is eluted in a volume of 200$\mu$l (in two steps, 100$\mu$l each) AE buffer. The concentration is determined via UV measurement at 260nm in a volume of 2$\mu$l using a Peqlab NanoDrop®1000 Spectrophotometer (Peqlab, Erlangen, Germany). The purity of the isolated DNA is determined by OD260/280 ratio.

Amplification and quantitative detection of the centromere-specific fragments of human chromosome 17 is performed using a custom-designed (AppliedBiosystems) primer-probe-assay targeting a region described by Warburton et al.

For RealTime PCR, the ready-to-use primer-probe-assay and the TaqMan® Universal PCR Master Mix, No AmpErase® UNG (Applied Biosystems GmbH, Weiterstadt, Germany, Prod-No. 4324018) were used. The PCR reaction and quantification is performed in a StepOnePlus™ RealTime PCR System (Applied Biosystems GmbH, Weiterstadt, Germany, Prod-No. 4376600) applying the standard protocol supplied by the manufacturer. For quantitative analysis a $\Delta$CT method was used. Genomic DNA from a human breast carcinoma xenograft (MaCa 3366) as a positive control, from NOD/SCID mouse liver as a negative control as well as a water sample as a reagent control are processed in parallel.

Data represents the mean of two tissue samples from one group of mice treated similarly, each determined in duplicate.

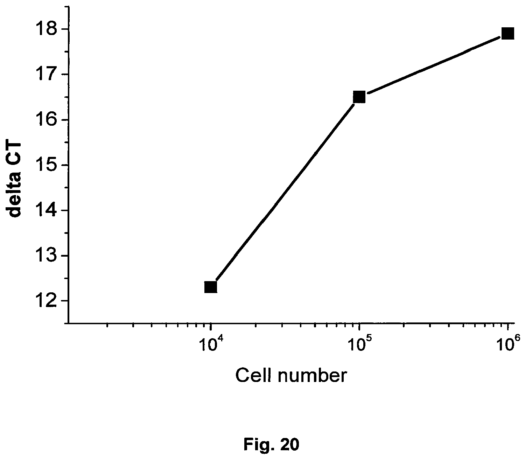

Table 3 demonstrates that there is a clear impact of cell number (see also figure 20) and time of experiment (not shown) on the degree of metastasis, which was directly proportional. Best results were obtained if $10^5$ cells were injected and mice were sacrificed after 20 days.

## 12. *in vivo* toxicity studies of CRA-entrapped nanocarriers

The Embodiment disclosed the subsequent Section 12 adress the unclaimed type of nanocarrier composed of polymeric nanoparticles, which are not covered by the claims.

An appropriate amount of freeze dried, Teijin loaded PNPs were resuspended in phosphate buffered saline, pH 7.5 and vortexed until a clear solution was obtained. This solution was injected immediately after preparation.

Three NSG mice / group (mean weight 16 g) were intravenously injected with a single dose between 2 and 250 mg/kg of Teijin-loaded polymeric nanoparticles. Mice were monitored and weighted daily until they were sacrificed at day 23. An autopsy was performed.

The PNPs were well tolerated at a dose between 2 - 125 mg/kg. Only the highest dose was toxic for one mouse and caused serious acute but transient toxic effects like convulsions, breathing and cardiovascular problems.

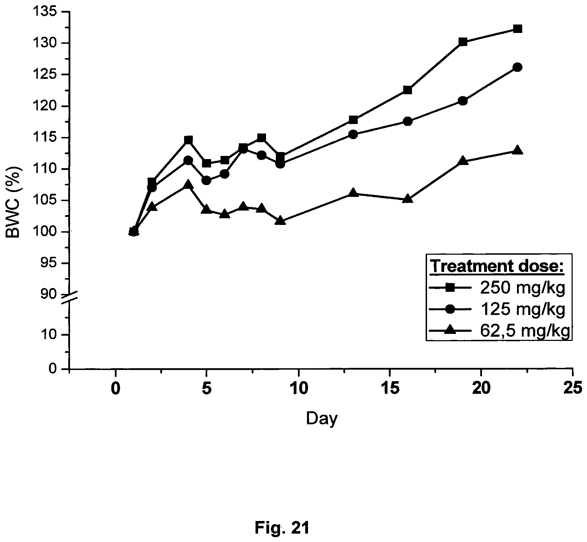

The body weight change diagram for highest doses demonstrated that no general toxic effects (observed as body weight loss) appeared (figures 21) up to a dose of 125 mg/kg. Treatment with a dose of 250 mg/kg was toxic for one mouse immediately after treatment. Autopsy at the end of the study revealed no gross organ changes.

That demonstrates that mice could be treated with a dose of up to 125 mgTeijin/kg given as a single i.v. injection. This dose defines the approximate dose which could be applied in therapeutic studies.

## **TABLES**

**Table 1:** Lipid composition of liposomes

| Code | Liposome composition | | | | |
|---|---|---|---|---|---|
| | DOPE (mol %) | DOPA (mol %) | PEG-DSPE (total mol % PEG) | Mal-PEG-DSPE (mol %) | PEG-DSPE (mol %) |
| TSL-1 | 60 | 35 | 5 | 2 | 3 |
| TSL-2 | 75 | 20 | 5 | 2 | 3 |
| TSL-3 | 85 | 10 | 5 | 2 | 3 |
| TSL-4 | 61 | 35 | 4 | 2 | 2 |
| TSL-5 | 62 | 35 | 3 | 2 | 1 |
| TSL-6 | 63 | 35 | 2 | 2 | 0 |
| TSL-7 | 64 | 35 | 1 | 1 | 0 |
| TSL-8 | 60 | 35 (DPPG) | 5 | 2 | 3 |

Composition of control liposomes CL-1 was: POPC: Chol: Mal-PEG-DSPE: PEG-DSPE, molar ratio 60:35:2:3.

**Table 2:** Parameters of Teijin compound 1 and FITC-BSA loaded nanoparticles (not covered by the claims)

| Form. | Active agent and amount (mg) | Polymer and concentration | Particle size (nm) | P.D. | Encapsulation efficiency (%) |
|---|---|---|---|---|---|
| 1 | Teijin comp. 1 mg | A-MA-PEG-RG502 2% | 206.3±9.2 | 0.168 | 19.6 |
| 2 | Teijin comp. 1 mg | A-MA-PEG-RG503 2% | 219.1±7.1 | 0.132 | 56.6 |
| 3 | Teijin comp. 1 mg | A-MA-PEG-RG504 2% | 226.2±9.8 | 0.186 | 59.1 |
| 4 | Teijin comp. 2 mg | A-MA-PEG-RG503 2% | 215.9±3.8 | 0.135 | 51.1 |
| 5 | Teijin comp. 2 mg | A-MA-PEG-RG503 2% | 218.2±6.1 | 0.120 | 92.0 |
| 6 | FITC-BSA 5 mg | A-MA-PEG-RG503 2% | 211.5±4.9 | 0.134 | 76.1 |

Size and loading efficacy were determined after peptide conjugation. PLGA nanoparticle (not covered) were prepared using the polymer in a concentration of 2%. The inner aqueous phase was 0,5 ml, while the organic phase consisted of 5 ml DCM. The inner phase sufactant PVA was added in 2,8%. Additional parameters: primary and secondary emulsion stirring rate was 9500rpm for 1.5 and 1.75 min, respectively; 0,4% PVA was added as outer phase surfactant.

**Table 3:** Impact of cell number on degree of metastasis in the LU 9313M lung carcinoma model

| Group | Cell number i.v./mouse | Liver weight [g] | Liver metastases | | PCR Delta CT*** |
|---|---|---|---|---|---|
| | | | n | scores | |
| A | $10^4$ | 1.73±0.45 | 3±3* | 12±10 | 12.3±2.0 |

(continued)

| Group | Cell number i.v./mouse | Liver weight [g] | Liver metastases n | scores | PCR Delta CT*** |
|---|---|---|---|---|---|
| B | $10^5$ | 1.88±0.45 | 15±1,4** | 140±22** | 16.5±1.8 |
| C | $10^6$ | 6.47±1.94 | n.c. | | 17.9±3.4 |

*Time point of determination: Day 20* *n.c.: not possible to count; liver was completely infiltrated with metastases*

*\*: One mouse was without any metastases*

*\*\*: One mouse with more than 62 metastases (corresponding to a score of 1734) was excluded from data summary*

*\*\*\*: Delta CT for positive sample of human tumor cells:18.3*

**FIGURE LEGENDS**

**Figure 1: Schematic presentation of the basic idea of the approach.** Nanoparticles, NP (TSL or not claimed PNP) bind to a specific target on EC's surface (i.e. VCAM-1) and release chemokine antagonists (CA) that are able to inhibit the interaction chemokines/chemokines receptors leading to a inhibition in transmigration of leukocytes or tumor metastatic cells.

**Figure 2: Morphology of targeted nanoparticle.** Scanning electron microscopy (SEM) photographs of (a) peptide coupled Teijin loaded and (b) FITC-BSA loaded modified PLGA (A-MA-PEG-RG503) nanoparticles (not claimed). Figure c shows the Transmission electron microscopy (TEM) photographs of (a) peptide coupled Teijin loaded liposomes. Liposomeswere stained with phosphotungstic acid (1%) and visualized by negative staining electron microscopy (scale bar: 100 nm).

**Figure 3: Release of calcein from target-sensitive liposomes TSL after storage in buffer.** (A): Release of calcein from target-sensitive liposomes TSL (DOPE: DOPA: PEG-DSPE: Mal-PEG) with 5 mol % PEG and different concentration of DOPA in the phospholipidic bilayer (A and B). Calcein release after storage in PBS for different time up to 50 days at 4°C. (B): Calcein release after incubation in PBS for time up to 24 hour at 37°C.

**Figure 4: Release of calcein from target-sensitive liposomes in the presence of serum.** Shown is the release of calcein in percent from target-sensitive liposomes (DOPE: DOPA: PEG-DSPE: Mal-PEG) composed of 35 mol% DOPA and different concentration of phospholipidic derivates of PEG (1 to 5 mol%) in the presence of 20 % (A) and 50 % fetal bovine serum (B). 5 mol% (TSL-1), 4 mol% (TSL-4), 3 mol% (TSL-5), 2 mol% (TSL-6) and 1 mol % PEG (TSL-7) were used.

**Figure 5: *In vitro* release profile of Teijin from targeted PEGylated PLGA nanoparticles (not claimed).** The release of Teijin from medium molecular weight targeted PLGA nanoparticle (Peptide-A-MA-PEG-RG503 and from high molecular weight targeted PLGA nanoparticle(Peptide-A-MA-PEG-RG504), loaded with 1 mg Teijin, in buffer were followed for 70 days.

**Figure 6: Cytotoxicity of empty targeted PEGylated PLGA nanoparticles (not claimed) on Human Vascular Endothelial Cells (HUVECs).**
7.5 x 103 cells were incubated with nanoparticles at concentrations from 0 mg/mL to 5mg/mL (0, 0.05, 0.1, 0.5, 1, 2 and 5 mg/mL) in PBS (pH 7.4) for 72 hours. The percentage of cell viability was determined by MTT assay relative to untreated control cells. Polyethylenimine, a polycation, was used as positive control. Shown are the mean values +/-S.D.(n=3).

**Figure 7: Binding of targeted TSL to activated endothelial cells.** (A) Flow cytometry chart showing the binding to human endothelial cells (EC) of fluorescently labelled non-targeted TSL-1 liposomes (blue line), targeted TSL-1 to non-activated (black line) or TNF-$\alpha$ activated human ECs (red line), and ECs only (green line). (B) Fluorescence microscopy of VCAM-1 targeted TSL-1 liposomes binding to activated EC in the absence and after preincubating the cells with 10 $\mu$g/ml soluble VCAM-1 recombinant protein (C). Red colour indicates liposomes and blue colour is given by nuclei stained with DAPI.

**Figure 8: Calcein release from targeted TSL-1 and control liposomes after interaction with non-activated or TNF-α activated human endothelial cells (EC).** Kinetic of calcein release from targeted TSL-1 with following composition DOPE: DOPA: PEG-DSPE: Mal-PEG at a molar ratio 60:35:2:3 mol%) after interaction with non-activated (targeted TSL-1+EC) or TNF-α activated (targeted TSL-1+aEC) human endothelial cells (EC) and from control, targeted non-sensitive liposomes (CL-1 coupled with peptide recognizing VCAM-1 with composition POPC:Chol: Mal-PEG-DSPE:DSPE-PEG at a molar ratio 60:35:2:3 mol%) after binding to the surface of activated EC (targeted CL-1 +aEC). Calcein release measured every 5 minutes in the first hour (A) and every hour up to 24 hours (B).

**Figure 9: Release of Teijin from targeted TSL-1, non-targeted TSL-1 and targeted non-sensitive liposomes (CL-1) after interaction with activated endothelial cells.** Percent of release of CCR2 antagonist (Teijin compound 1) entrapped into targeted TSL-1, non-targeted TSL-1 and targeted non-sensitive liposomes (CL-1) 1 on human monocytes (isolated from peripheral blood) adhesion to activated endothelial cells evaluated using the fluorescence microscope to determine the number of fluorescently labeled monocytes that adhered to endothelial cells. The number of adhered monocytes was determined and the percent inhibition of monocyte adhesion to activated EC in the presence of free or liposome-encapsulated Teijin was calculated relative to the control (monocytes incubated with activated cells in the absence of Teijin).

**Figure 10: Release of calcein from targeted or non-targeted TSL-1 after interaction with immobilized VCAM-1 protein under flow conditions.** (A) Release of calcein from targeted or non-targeted TSL-1after interaction with VCAM-1 protein immobilized on coverglasses under flow conditions for a period of 24h. (B) Release of calcein from targeted TSL-1after binding to EC non-activated or activated with TNFα. (C) Release of calcein from TSL-8 target-sensitive liposomes (DOPE: DPPG: PEG-DSPE: Mal-PEG at a molar ratio 60:35:3:2 mol%) after interaction with VCAM-1 protein immobilized on coverglasses under flow conditions for periods up to 24 h.

**Figure 11: Inhibition of adhesion of human monocytes to activated endothelial cells by free or liposomally entrapped Teijin in vitro.** The inhibitory effect of different concentration of Teijin compound 1, free or entrapped in targeted TSL-1, non-targeted TSL-1, or targeted non-sensitive liposomes CL-1 on human monocytes (isolated from peripheral blood) adhesion to activated endothelial cells (isolated from peripheral blood) adhesion to activated endothelial cells cells was evaluated using the fluorescence microscope to determine the number of fluorescently labelled monocytes that adhered to endothelial cells. The number of adhered monocytes was determined and the percent inhibition of monocyte adhesion to activated EC in the presence of free or liposome-encapsulated Teijin was calculated.

**Figure 12: Binding of VCAM-1 targeted (A) and non-targeted (C) TSL-1 to VCAM-1 immobilized on the gold sensor surface.** The binding of VCAM-1 targeted (A) and non-targeted (C) TSL-1 to VCAM-1, immobilized on the gold sensor surface, was folloed by measuring the amplitude as indicator for surface viscoelasticity. The initial increase of the amplitude (red arrows) indicate a surface rigidification, mediated by a spreading of the TSL (B). This could not be obtained using conventional liposomes (targeted CL-1).

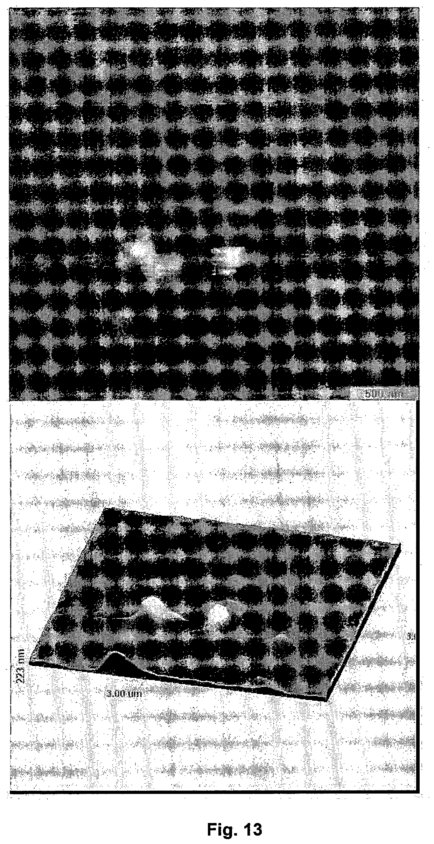

**Figure 13: Immobilization of targeted TSL a VCAM-1 immobilized at the surface of a gold sensor.** SAW sensor with VCAM-1 immobilized on the surface and incubated with targeted TSL-1 liposomes (0.1μM). ). Note the 2D image on the left panel and the 3D image on the right panel. Image size is 3X3um in SPFM AC mode with 5V at 3kHz. Both images show a broken liposome after the adhesion to VCAM-1 next to one that has remained intact.

**Figure 14: Volume measurements of the liposomes.**
The volume was measured from the SPFM topography images by integration under the marked area, the volume between the liposome surface and the plane z = $z_{min}$, where $z_{min}$ is the minimum value of height occurring in the marked area. This method accounts for grain surrounding and measures only the volume above the surface of the sensor;

A) on the right the non-collapsed liposome marked for measurement;
B) on the left the area containing the collapsed liposome marked for volume measurement. The area for this measurement was marked by excluding the previous marked area i.e. of the non-collapsed liposome from the entire region with height above $z_{min}$, the height of the surface of the sensor

**Figure 15. Interaction of targeted TSL-1 with BSA immobilized at the surface of a gold sensor.** Pictures show the hydrated BSA-covered sensor before (left) and after adding of targeted TSL-1 liposomes (0.01μM, right). Image size is 3X3 μm in SPFM AC mode with 5V at 3kHz

**Figure 16.** Effect of Teijin compound 1 either free or entrapped in targeted TSL-1, non-targeted TSL-1, or targeted CL-1 on human monocytes transmigration.

Then inhibition of transmigration of human monocytes (isolated from peripheral blood) by CCR2 antagonist, Teijin compound 1 either free or entrapped in targeted TSL-1, non-targeted TSL-1, or targeted CL-1 was evaluated using a fluorescence plate reader. The percent inhibition of monocyte transmigration in the presence of free or liposome-encapsulated Teijin was calculated relative to the control (monocytes incubated with activated cells in the absence of free or liposome-encapsulatedTeijin).

**Figure 17: Binding of Rhodamin-PE labelled targeted liposomes to aortas of ApoE-deficient mice after *in situ* administration.** (A) Binding of Rhodamin-PE labelled non-targeted (Aa) or targeted CL-1 (Ab) to ApoE-deficient mice aortas after in situ administration into the aortas were visualized using IVIS imaging system Caliper 200 at λex = 535 nm and λem = 620 nm. (Ac) presents the aorta of an ApoE-deficient mouse after administration of PBS instead of liposomes. (B) Spectral mixing performed using Living image software shows in green tissue auto-fluorescence and in red specific fluorescence given by liposomes bound to aorta.

**Figure 18: Binding of Rhodamin-PE labelled targeted liposomes to aortas of ApoE-deficient mice after in situ administration.** (A) Binding of Rhodamin-PE labelled non-targeted (a) or targeted CL-1 (b) to ApoE-deficient mice aortas after in situ administration into mice aortas were visualized using IVIS imaging system Caliper 200 at λex = 535 nm and λem = 620 nm. (c) presents the aorta of an ApoE-deficient mouse after administration of PBS instead of liposomes. (B) Spectral unmixing performed using Living image software shows in green tissue auto-fluorescence and in red specific fluorescence given by liposomes bound to aorta.

**Figure 19. The treatment with Teijin-encapsulated TSL-1 reduces atherosclerotic lesion development in ApoE-deficient mice.** (A) Oil Red O stained *en face* preparations of aorta and (B) quantitative data. *p<0.05 vs. control.

**Figure 20: Impact of cell number on on degree of metastasis in the LU 9313M lung carcinoma model.** NOD/SCID mice were injected i.v. with the indicated cell numbers and sacrificed after 20 day. Lung were isolated and rtPCR was performed to quantify the amount of human cancer cell in the mouse lung as measure for metastasis.

**Figure 21: Tolerability of Teijin loaded targeted PEGylated PLGA nanoparticles (not claimed) by mice.** Female NSG mice (3 mice/group) were injected intravenously with with a single dose of Teijin loaded targeted PEGylated PLGA nanoparticles and body weight change (BWC) was followed for 25 days. BWC in % was calculated in relation to that at treatment day (day 0).

## Claims

1. Nanocarrier for targeted drug delivery to inflammatory sites, **characterized in that** the nanocarrier are

   • liposomes comprising dioleoylphosphatidylethanolamine (DOPE) stabilized in a bilayer with dioleoylphospatidicacid (DOPA) or 1,2-dipalmitoyl-*sn*-glycero-3-phospho-(1'-*rac*-glycerol) (DPPG)
   • and wherein said nanocarrier comprising a peptide or antibody which specifically binds to endothelial cell adhesion molecule VCAM-1
   • and wherein said nanocarrier encapsulate chemokine antagonists (CA) or chemokine receptor antagonists (CRA).

2. Nanocarrier according to claim 1, **characterized in that** the liposomes comprises polyethyleneglycol (PEG) modified phospholipids.

3. Nanocarrier according to Claim 1, **characterized in that** the VCAM-1 specific peptide is coupled to the distal end of the polyethyleneglycol .

4. Nanocarrier according to claim 1 **characterized in that** Chemokine (C-C motif) Ligand 2 Antagonist (CCL2-Antagonist) or Chemokine (C-C motif) Ligand 5 Antagonist (CCL-5-Antagonist) is encapsulated.

5. Nanocarrier according to claim 1, **characterized in that** the Chemokine Receptor 2 Antagonist Teijin or the Chemokine Receptor 5 Antagonist DAPTA is encapsulated.

**6.** Nanocarrier according to any one of the claims 1-6, **characterized in that** they have a particle size between 50 and 500 nm, preferably between 70 and 200 nm, more preferably 200 nm.

**7.** Nanocarrier according to any one of the Claims 1-7 for use in blocking cancer cell metastasis.

**8.** Nanocarrier according to any one of the Claims 1-8 for use in blocking inflammatory processes in vascular diseases.

**Patentansprüche**

**1.** Nanocarrier für die gezielte Wirkstoffverteilung an Entzündungsorten, **dadurch gekennzeichnet, dass** die Nanocarrier bestehen aus

• Liposomen aus Dioleoylphosphatidylethanolamin (DOPE) stabilisiert in einer Doppelschicht mit Dioleoylphospatidicacid (DOPA) oder 1,2-Dipalmitoyl-*sn*-glycero-3-phospho-(1'-*rac*-glycerol) (DPPG),
• und worin diese Nanocarrier ein Peptid oder Antikörper mit spezifischer Bindung zu einem endothelialen Zell-Adhäsionsmolekül VCAM-1 sind
• und worin diese Nanocarrier Chemokin Antagonisten (CA) oder Chemokin Rezeptor Antagonisten (CRA) einkapseln.

**2.** Nanocarrier gemäß Anspruch 1, **dadurch gekennzeichnet, dass** die Liposomen Polyethylenglycol (PEG) modifizierte Phospholipide sind.

**3.** Nanocarrier gemäß Anspruch 1, **dadurch gekennzeichnet, dass** das VCAM-1 spezifische Peptid am entfernten Ende des Polyethylenglycols gekoppelt ist.

**4.** Nanocarrier gemäß Anspruch 1, **dadurch gekennzeichnet, dass** Chemokin (C-C Motiv) Ligand 2 Antagonist (CCL2-Antagonist) oder Chemokin (C-C Motiv) Ligand 5 Antagonist (CCL-5-Antagonist) eingekapselt ist.

**5.** Nanocarrier gemäß Anspruch 1, **dadurch gekennzeichnet, dass** der Chemokin Rezeptor 2 Antagonist Teijin oder der Chemokin Rezeptor 5 Antagonist DAPTA eingekapselt ist.

**6.** Nanocarrier gemäß einem der Ansprüche 1-6, **dadurch gekennzeichnet, dass** sie eine Partikelgröße zwischen 50 und 500 nm, bevorzugt zwischen 70 und 200 nm, besonders bevorzugt 200 nm, aufweisen.

**7.** Verwendung der Nanocarrier gemäß einem der Ansprüche 1-7 zur Blockierung von Krebszellmetastasen.

**8.** Verwendung der Nanocarrier gemäß einem der Ansprüche 1-8 zur Blockierung von entzündlichen Prozessen bei Gefäßerkrankungen.

**Revendications**

**1.** Nanocarrier pour la libération ciblée de médicaments dans des foyers inflammatoires, **caractérisé par le fait que** les nanocarriers sont

• des liposomes comprenant de la dioléoylphosphatidyléthanolamine (DOPE) stabilisée dans une bicouche avec de l'acide dioléoylphospatidique (DOPA) ou du 1,2-dipalmitoyl-*sn*-glycéro-3-phospho-(1'-*rac*-glycérol) (DPPG)
• et dans lesquels lesdits nanocarriers comprennent un peptide ou un anticorps qui se lie spécifiquement à la molécule d'adhésion des cellules endothéliales VCAM-1
• et dans lesquels lesdits nanocarriers encapsulent des antagonistes de chimokines (CA) ou des antagonistes de récepteurs de chimokines (CRA).

**2.** Nanocarrier conformément à la revendication n°1, **caractérisé par le fait que** les liposomes comprennent des phospholipides modifiés par du polyéthylèneglycol (PEG).

**3.** Nanocarrier conformément à la revendication n°1, **caractérisé par le fait que** le peptide spécifique VCAM-1 est couplé à l'extrémité distale du polyéthylèneglycol.

4. Nanocarrier conformément à la revendication n°1, **caractérisé par le fait que** l'antagoniste Ligand 2 de la chimokine (C-C motif) (antagoniste CCL2) ou l'antagoniste Ligand 5 de la chimokine (C-C motif) (antagoniste CCL-5) est encapsulé.

5. Nanocarrier conformément à la revendication n°1, **caractérisé par** le fait l'antagoniste du récepteur de type 2 de la chimokine Teijin ou l'antagoniste du récepteur de type 5 de la chimokine DAPTA est encapsulé.

6. Nanocarrier conformément à l'une des revendications n°1 à n°6, **caractérisé par le fait que** les nanocarriers ont une taille de particule comprise entre 50 et 500 nm, de préférence entre 70 et 200 nm, idéalement de 200 nm.

7. Nanocarrier conformément à l'une des revendications n°1 à n°7 pour une utilisation visant à bloquer les métastases cancéreuses.

8. Nanocarrier conformément à l'une des revendications n°1 à n°8 pour une utilisation visant à bloquer les processus inflammatoires dans les maladies vasculaires.

Fig. 1

**Fig. 2**

Fig. 3

**Fig. 4**

**Fig. 5**

**Fig. 6**

(A)

(B)                    (C)

**Fig. 7**

Fig. 8

Fig. 9

**A** DOPE:DOPA:DSPE-PEG:MalPEG (60:35:3:2 mol%)

- TSL-VCAM-1 peptide
- TSL

% carboxyfluorescein release vs time (hours)

**B**

- TNF activated cells
- non-activated cells

% carboxyfluorescein release vs time [hours]

**C** DOPE:DPPG:DSPE-PEG:MalPEG (60:35:3:2 mol%)

- TSL-VCAM-1 peptide
- TSL

% carboxyfluorescein release vs time [hours]

**Fig. 10**

**Fig. 11**

31

**Fig. 12**

**Fig. 13**

**(A)**

**(B)**

**Fig. 14**

**Fig. 15**

**Fig. 16**

A

B

**Fig. 17**

**Fig. 18**

**Fig. 19**

**Fig. 20**

**Fig. 21**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6451842 B1
- WO 9320102 A1
- DE 4324018
- DE 4376600

### Non-patent literature cited in the description

- **SIMIONESCU M.** *Arterioscler. Thromb. Vasc. Biol.,* 2007, vol. 27, 266-74
- **ROT, A. ; VON ANDRIAN U.H.** *Annu. Rev. Immunol.,* 2004, vol. 22, 891-928
- **MANTOVANI A. et al.** *Nature,* 2008, vol. 454, 436-44
- **LAUBLI H. et al.** *Blood,* 2009, vol. 114, 4583-91
- **PROUDFOOT A.E. ; POWER C.A. ; SCHWARZ, M.K.** *Expert Opin Investig Drugs,* 2010, vol. 19, 345-55
- **MOREE W.J. et al.** *Bioorganic and medical chemistry letters,* vol. 18 (6), 1869-1873
- **DANHIER F. et al.** *J. of controlled release,* vol. 161 (2), 505-522
- **SIMONE E. et al.** *Cell and tissue research,* vol. 335 (1), 283-300
- **MOREOVER, BRECHT A. et al.** *Regulatory Peptides,* vol. 166 (1-3), 76-82
- **PREISS D.J. et al.** *Int. J. of Clinical Practise,* vol. 61 (4), 697-701
- **MURO S.** *J Control Release,* 2012, vol. 164, 125-37
- **KUMAR P ; GULBAKE A ; JAIN SK.** *Crit Rev Ther Drug Carrier Syst.,* 2012, vol. 29, 355-419
- **SWAMINATHAN J ; EHRHARDT C.** *Expert Opin Drug Deliv.,* 2012, vol. 9, 1489-503
- **PATERNOSTRE M. et al.** Manual on Membrane Lipids. Springer-Verlag, 1996, 218Y226